# EUROPEAN PATENT SPECIFICATION

(11) **EP 3 914 177 B1**
(45) Date of publication and mention of the grant of the patent: **20.05.2026**
(21) Application number: 20744615.4
(22) Date of filing: 23.01.2020
(51) Int. Cl.: A61B 18/14, A61M 5/178

(54) **DEVICES FOR ABLATING TISSUE**
VORRICHTUNGEN ZUR ABLATION VON GEWEBE
DISPOSITIFS POUR L'ABLATION DE TISSU

(30) Priority: 23.01.2019 AU 2019900214
(43) Date of publication of application: 01.12.2021
(73) Proprietor: Ablation Gen 2 Pty Ltd, Malvern VIC 3144 (AU)
(72) Inventor: MORRIS, David, Kogarah, NSW 2217 (AU); MORRIS, Christopher, deceased (AU); ALTOUKHI, Khaled, Kogarah, NSW 2217 (AU); VALLE, Sarah, Kogarah, NSW 2217 (AU)
(74) Representative: Patent Boutique LLP
(86) International application number: PCT/AU2020/050043
(87) International publication number: WO 2020/150782

(56) References cited:
- WO-A1-2017/074901
- WO-A1-96/04860
- WO-A2-00/13602
- US-A1- 2008 071 264
- US-A1- 2008 167 649
- US-A1- 2011 077 644
- US-A1- 2011 077 644
- US-A1- 2014 276 764

## Description

### Technical Field

The present invention relates to devices, methods and systems for ablating tissue. In specific embodiments, the present invention relates to a device according to claim 1 for ablating lung and other soft tissue tumours.

### Background Art

Tumours found in many body organs are often not able to be surgically removed and it is therefore necessary to treat the tumour *in situ.* A number of techniques are known for such *in situ* treatments, including techniques that use radio frequency (RF) to generate heat which is capable of ablating biological tissue in proximity to the electrode of an ablation device that has been inserted into the patient. In effect, the generated heat kills the tumour cells.

In use, RF ablation devices are inserted into the target tissue (often directly into a tumour) and operated to ablate surrounding tissue upon application of an electrical field, either between the device's electrode(s) and a grounding pad positioned on the patient's skin (in the case of a monopolar ablation device) or between electrodes of the device having an opposite polarity (in the case of a bipolar ablation device). The use of such ablation devices for treating tumours in some body organs can, however, be problematic.

For example, whilst the ablation of primary and secondary lung cancers is now widely practiced and is a minimally invasive technique that can have outcomes comparable to those of surgical resections (but with considerably less morbidity, hospitalisation costs and adverse effects), such ablation procedures have an inherent risk due to the necessity to pierce the patient's lung in order to access and ablate the tumour. Pneumothorax (leakage of air from the lung into the pleural space) is the most frequent complication of percutaneous lung ablations. When such occurs, further surgical intervention would usually be required in order to insert a chest drain. This can be a painful process and carries risks of inadvertent damage to intra-thoracic organs including the lungs, large blood vessels, oesophagus and even the heart. An untreated pneumothorax can delay discharge from hospital and might also cause respiratory difficulty and even death, particularly if a tension pneumothorax were produced.

Complications can also arise when ablating tumours in some organs because it is often necessary to use smaller ablation devices and/or to insert the devices into the tumour in order to achieve an effective ablation. Such devices either may not be able to produce a tissue ablation having a predictable volume and size (precision and predictability of ablations is important both to protect body parts surrounding the tumour as well as to completely destroy the tumour) or may carry a risk of tumour seeding, where malignant cells are spread along the track of the device during its withdrawal.

Relevant prior art is disclosed in US 2011/077644 Al, US 2008/167649 A1, US 2014/276764 A1 and US 2008/071264 A1.

### Summary of Invention

The invention is defined by appended independent claim 1. Further embodiments are disclosed in the dependent claims. Methods of treatment are disclosed for illustrating the workings of the device and are not claimed.

In a first aspect, the present invention provides a tissue ablation device. The device comprises a sheath comprising a distal end that is positionable at an ablation site in the tissue, a proximal end and a lumen extending therebetween. The device also comprises one or more electrodes that are advanceable and retractable through the lumen. A distal portion of the one or more electrodes is deployable into a tissue ablating configuration from the distal end of the sheath upon advancement, and the one or more electrodes are removable from the lumen via the proximal end of the sheath upon retraction, whereupon the lumen becomes vacated. The sheath is configured for a surgical material to traverse the vacated lumen for delivery from the distal end of the sheath into the tissue.

As will be described in further detail below, the inventors have discovered that a device configured for the delivery of a surgical material or materials directly into the ablation site (and/or the track along which the device's sheath has travelled in order to reach the ablation site) using the device's already *in situ* sheath can significantly reduce the risk of side effects when ablating biological tissue (or when inserting or withdrawing the device's sheath), even in body locations that are conventionally relatively high risk. For example, if a surgeon or interventional radiologist were to remove a conventional ablation device and then attempt to place a second device into the track in order to block an air leak or to stem bleeding (for example), this would be unlikely to be successful because of the high likelihood of missing the intended track. It would, for example, be very difficult to follow the same track as the ablation device when ablating lung tumours, as the lung is constantly moving and not attached to the chest wall where the needle was inserted. In the meantime, air or blood will have begun to leak from the tissue and track which, once started, is more difficult to control. In contrast, the device of the present invention allows immediate access to the tissue and track formed during insertion of the sheath, through the vacated lumen of the *in situ* sheath.

Surgical materials may, for example, include a tissue sealant for injection into the track during removal of the sheath, which could help to prevent conditions such as pneumothorax. Alternatively, or in addition, a chemotherapeutic agent could be delivered into the ablated tumour site, and then the track as the sheath is withdrawn, in order to kill any remaining malignant cells and prevent them from seeding. Alternatively, or in addition, a pro-coagulant could be delivered into the ablated tumour site and then the track as the sheath is withdrawn, in order to control bleeding. Alternatively, or in addition, a device capable of occluding the track in order to prevent air from leaking (i.e. when ablating lung tumours) or excessive bleeding could be delivered (either directly or indirectly, as discussed below) via the vacated lumen.

The inventors recognised that conventional ablation devices, in which the electrodes are integral to the device and are thus not readily removable, would simply not be capable of being used to deliver many surgical materials. Whilst flowable surgical materials might be able to be delivered through a lumen provided in such devices, its flow through the lumen would not only be hindered by the electrodes (and any other components), but its delivery into the patient may be adversely affected in any number of unpredictable ways. For example, the flowable surgical material may flow out of the lumen at too high a pressure and distribute irregularly and/or emit from the lumen's distal end at an undesirable angle. Further, in embodiments where the surgical material is a tissue sealant, any electrodes or other components present in the lumen could become permanently fixed therein. In contrast, the tissue ablation device of the present invention enables its operator to deliver the surgical material or materials in a controlled and predictable manner.

The ablation device of the present invention may thus advantageously reduce the risks associated with the treatment of tumours in conventionally problematic areas of the body. Even if complications were to occur during insertion. ablation or withdrawal, the device's *in situ* sheath provides a means via which many of such complications could be quickly addressed (or pre-empted), thereby reducing the likelihood of the need for further surgical intervention. For example, embodiments of the ablation device for use in ablating lung tumours that can perform both ablation and seal the hole between the lung and pleural cavity have significant potential to prevent the occurrence of pneumothorax or to control bleeding. As noted above, inserting a second, independent, device to seal the track following ablation would be very difficult, as the lung is constantly moving and not attached to the chest wall where the needle was inserted. Furthermore, repeated insertion of instruments would lengthen the time needed to complete the procedure and would disturb natural coagulation.

In some embodiments, the device may further comprise a dispenser which is operable to dispense the surgical material into the vacated lumen. The dispenser may, for example, be attachable to the proximal end of the sheath.

In some embodiments, the one or more electrodes may be configured to have a polarity during ablation opposite that of a grounding pad positioned on the body of a patient, whereby ablation occurs between the electrode(s) and the grounding pad. In some embodiments, the one or more electrodes may be configured to have a polarity during ablation opposite that of an electrically conductive portion of the sheath, whereby ablation occurs between the electrode(s) and the electrically conductive portion of the sheath.

In some embodiments, the one or more electrodes may comprise a plurality of electrodes configured to have opposite polarities during ablation.

As will be described below, providing the various electrically active portions of the ablation device, and optionally a grounding pad positioned on a patient's skin in an appropriate location, with different polarities can result in potentially larger and more consistent and precise ablations. Such embodiments may also enable more effective ablations when treating tumours in organs such as the lungs which, as described below, are complicated because of the presence of unconducive air pockets in and around the tumour.

In the invention, the electrode(s) may be configured to assume a predetermined shape upon deployment into the tissue ablating configuration. The tissue ablating configuration of the electrode(s) is circular or helical in shape, which the inventors have found can result in a relatively longer lengths of electrode being deployable into the tissue. The inventors have found that the length of the electrode deployed into the tissue is generally proportional to the amount of electrical current that can be applied, and hence longer deployed electrodes generally correlates with faster and/or larger ablations.

In some embodiments, the electrode(s) may have a cross-sectional shape that is independently selected from: circular, a circular segment, elliptical, triangular and flat. Electrodes having such shapes may have beneficial properties such as strength and reliable deployment characteristics. Surface area for electrical conduction and heat dissipation are also considerations relevant to the cross sectional shape of the electrodes.

In some embodiments, the sheath may be configured for percutaneous insertion (e.g. through a patient's chest cavity). In other embodiments, the sheath may be configured for endoscopic insertion (e.g. via the patient's airways using a bronchoscope).

In the invention, the distal end of the sheath is configured by being crenulated to guide an initial direction of deployment of the one or more electrodes. Such guidance would tend to provide more consistent and reliable deployment configurations, which would be important when ablation is to be carried out in close proximity to vital organs, for example. Such crenulations may also be sharpened in order for the sheath to more easily pierce tissue. An interior of the sheath may also include grooves, indentations or ribs (provided that such do not affect the functionality of the vacated lumen) which can also help to guide deployment of the electrode(s).

In some embodiments, the ablation device may further comprise a deployment handle which is operable to advance and retract the one or more electrodes through the lumen. The deployment handle may, for example, be removably attachable to the tissue ablating device. The deployment handle may, for example, be orientated at an angle to the sheath and/or comprise a flexible portion or a cable-like portion. Such embodiments may be beneficial in situations where access to a patient is physically restricted, as would be the case, for example, if the device is being guided into position using real-time computerized tomography ("CT") or a MRI scanner.

In some embodiments, the electrode(s) may be deployable from the distal end of the sheath with an angle of deployment that is selectable by orientating the electrodes with respect to the sheath. In such embodiments, multiple ablations for each sheath insertion may be achieved simply by changing the angle of deployment of the electrode(s) from the distal end of the sheath (i.e. by rotating the device's electrodes with respect to its sheath) between ablations. The combined effect of the multiple ablations can produce a volume of ablated tissue that is greater than would be possible otherwise (i.e. without having to physically withdraw and reinsert the device at a new location). As such, fewer electrodes (and/or smaller electrodes) are required which, in turn, enables relatively thinner sheathes to be used. As would be appreciated, the thinner the sheath of an ablation device, the less invasive the ablation procedure. As would also be appreciated, minimising the number of times an ablation device needs to be inserted into a patient's body will also lead to simpler and less invasive procedures. Such embodiments are described in detail in co-pending International (PCT) patent application no. PCT/AU2019/050880, entitled DEVICES AND METHODS FOR ABLATING BIOLOGICAL TISSUE.

In a second aspect, the present disclosure provides an exemplary and unclaimed method for ablating tissue at an ablation site in a patient's body. The method comprises:
providing a device for ablating tissue, the device comprising:
   a sheath comprising a distal end that is positionable at an ablation site in the tissue, a proximal end and a lumen extending therebetween;
   one or more electrodes that are advanceable and retractable through the lumen, wherein a distal portion of the one or more electrodes is deployable into a tissue ablating configuration from the distal end of the sheath upon advancement, and the one or more electrodes are removable from the lumen via the proximal end of the sheath upon retraction, whereupon the lumen becomes vacated; and
   wherein the sheath is configured for a surgical material to traverse the vacated lumen for delivery from the distal end of the sheath into the tissue;
positioning (e.g. percutaneously or endoscopically) the distal end of the sheath in the patient at the ablation site;
deploying the one or more electrodes into the tissue ablating configuration and ablating tissue at the ablation site;
retracting the one or more electrodes back into the sheath and subsequently removing the one or more electrodes from the lumen; and
delivering the surgical material into the patient via the vacated lumen.

In the method, the vacated lumen of the ablation device's sheath provides a conduit directly into the ablation, as well as the and track made by inserting the sheath into the patient. The existence of such a conduit may provide a number of advantages, some of which were described above. Possibly one of the primary advantages of this method, however, is that it may obviate any need for further surgical intervention (e.g. a second incision in the patent) in the event of complications such as bleeding and pneumothorax, because the lumen provides direct access to the ablation site and track for the introduction of a surgical material (e.g. substances or devices) to immediately treat complications such as bleeding and/or air leaks.

In some embodiments, the surgical material may be a flowable (e.g. liquid) surgical material. In such embodiments, the surgical material may be delivered into the patient by dispensing into the proximal end of the sheath (e.g. using a dispenser containing the flowable substance). Once so dispensed, the flowable surgical material flows through the vacated lumen, out of the sheath's distal end and into the patient's body. Examples of flowable surgical materials include tissue glues (e.g. for sealing holes between the lung/pleural cavity or preventing bleeding), chemotherapeutic agents (e.g. for killing any tumour cells that might have survived ablation, both at the ablation side and in the track) and pro-coagulants (e.g. for preventing or slowing bleeding).

In some embodiments, the surgical material may be a non-flowable surgical material, which is delivered into the patient by being dispensed into the proximal end of the sheath and pushed through the vacated lumen until it exits from the sheath's distal end and into the patient's body. Examples of non-flowable surgical materials include pleural drains, plugs, occluding devices (e.g. for physically plugging holes) or guide wires (that can be used to subsequently guide other items into and through the track).

In some embodiments, the surgical material may be dispensed whilst the sheath is being withdrawn out of the patient in order for the surgical material to be dispensed in an appropriate location (e.g. at a desired position in the track along which the sheath was inserted).

In a third aspect, the present disclosure provides an exemplary and unclaimed method for ablating a lung tumour in a patient. The method comprises:
providing a device for ablating lung tumours, the device comprising:
   a sheath comprising a distal end that is positionable at the lung tumour, a proximal end and a lumen extending therebetween;
   one or more electrodes that are advanceable and retractable through the lumen, wherein a distal portion of the one or more electrodes is deployable into a tissue ablating configuration from the distal end of the sheath upon advancement, and the one or more electrodes are removable from the lumen via the proximal end of the sheath upon retraction, whereupon the lumen becomes vacated; and
   wherein the sheath is configured for a surgical material to traverse the vacated lumen for delivery from the distal end of the sheath into the tissue;
percutaneously positioning the distal end of the sheath at the lung tumour;
deploying the one or more electrodes into the tissue ablating configuration and ablating tissue that includes the lung tumour;
retracting the one or more electrodes back into the sheath and subsequently removing the one or more electrodes from the lumen; and
dispensing tissue glue (or, in an alternative aspect, an occluding device) into and through the vacated lumen as the sheath is partially withdrawn out of the patient, whereby a hole between the patient's lungs and pleural cavity is sealed.

In some embodiments, the method may further comprise passing a pleural drain through the vacated lumen when the distal end of the sheath is located within the pleural cavity of the patient. The pleural drain would remain in the patient after the sheath has been fully withdrawn out of the patient. Thus, the required drain can be placed into the patient without the need for further surgical intervention which, as noted above, can result in a number of complications.

In some embodiments, the pleural drain may be larger than the shaft and hence not deliverable into the patient's body via the vacant lumen. In such embodiments, the method may further comprise passing a guide wire for a pleural drain through the vacated lumen when the distal end of the sheath is located within the pleural cavity of the patient. The guide wire remains in the patient after the sheath has been fully withdrawn out of the patient, where it can subsequently be used to guide a pleural drain into the patient's body along the track left by the ablation device's sheath using a conventional needle/wire/dilator procedure.

The present inventors have also discovered a unique ablation method which they expect can be used to enable relatively small ablation devices to produce larger and more controlled ablations than are achievable using conventional ablation devices and techniques, even in organs such as the lungs where ablation is complicated by the presence of non-conductive air. Thus, in a fourth aspect, the present disclosure provides an exemplary and unclaimed method for ablating tissue at an ablation site in a patient's body. The method comprises:
providing a device for ablating tissue, the device comprising:
   a sheath comprising a distal end that is positionable at an ablation site in the tissue, a proximal end and a lumen extending therebetween; and
   one or more electrodes that are advanceable and retractable through the lumen, wherein a distal portion of the one or more electrodes is deployable into a tissue ablating configuration from the distal end of the sheath upon advancement;
positioning the distal end of the sheath in the patient at the ablation site, and a grounding pad on the patient's body;
deploying the one or more electrodes into the tissue ablating configuration in the ablation site; and
causing ablation to occur wherein, during ablation:
   at least one of the one or more electrodes is caused to have a polarity that is opposite to that of the grounding pad, and
   an electrically conductive portion of the sheath or another of the one or more electrodes is caused to have a polarity that is the same as that of the grounding pad,
   whereby ablation progresses according to a relative impedance of tissue at the ablation site.

In effect, the unique method of the fourth aspect provides multiple electric pathways for the applied current to follow, which the inventors have discovered can advantageously result in ablations having relatively larger volumes and with consistent and predictable shapes. Such ablations are simply not achievable using conventional ablation devices.

The "monopolar/bipolar hybrid ablation method" described herein may advantageously be capable of effectively treating all tumours, and especially those in the lungs and in other body locations that have conventionally been relatively difficult to access and ablate. Operation of the hybrid ablation system described herein may also advantageously enable issues specific to the treatment of lung tumours (where the presence of air in close proximity to the tumour can affect the size and shape of the ablation due to air providing a high resistance to the path of the RF current) to be overcome.

In some embodiments, the methods of the second and third aspects may incorporate the ablation method of the fourth aspect. In some embodiments of the method of the second or third aspect, for example, the method may further comprise:
positioning a grounding pad on the patient, wherein during ablation:
at least one of the one or more electrodes is caused to have a polarity that is opposite to that of the grounding pad, and
an electrically conductive portion of the sheath or another of the one or more electrodes is caused to have a polarity that is the same as that of the grounding pad,
whereby ablation progresses according to a relative impedance of tissue at the ablation site.

In some embodiments, the ablation site may comprise or be a tumour in the patient's lung, pancreas, liver, thyroid, kidney, uterus, brain or breast. The present invention is expected to be well suited to treating such tumours, as some tumours in these organs can be relatively small (less than 2cm in diameter) and many of these organs are located in areas of the body that are inconsistent with the use of larger ablation devices. Furthermore, tumours in these organs tend to be in close proximity to vital structures due to limited space and a precise and carefully controlled ablation is therefore of great importance in order to avoid damaging adjacent structures such as the laryngeal nerve and pancreatic duct, whilst still achieving complete ablation of the tumour.

In some embodiments, the device for ablating tissue used in the method of the second, third or fourth aspect may be the tissue ablation device of the first aspect of the present invention, adapted as necessary based on the teachings contained herein, to achieve its required functionality.

Additional features and advantages of the various aspects of the present invention will be described below in the context of specific embodiments. It will be appreciated, however, that such additional features may have a more general applicability in the present invention than that described in the context of these specific embodiments.

### Brief Description of Drawings

Embodiments of the present invention will be described in further detail below with reference to the accompanying drawings, in which:
Figure 1 shows a tissue ablation device in accordance with an embodiment of the present invention with its electrodes in their tissue ablating configuration;
Figure 2 shows the ablation device of Figure 1, from which the electrodes have been removed;
Figure 3 shows enlarged views of the distal end of the sheath of the ablation device of Figure 1 and its deployed electrodes;
Figure 4 shows a cross sectional view of the sheath of the ablation device of Figure 1;
Figure 5 shows an enlarged view of the distal end of the sheath and deployed electrodes of a tissue ablation device in accordance with another embodiment of the present invention;
Figure 6 shows the ablation device of Figure 1 being used to deliver tissue glue via the sheath's vacated lumen into the lung and pleural cavity of a patient;
Figure 7 shows the ablation device of Figure 1 being used to deliver a pleural drain via the sheath's vacated lumen into the pleural cavity of a patient;
Figure 8 shows the ablation device of Figure 1 being used to deliver a guide wire via the sheath's vacated lumen into the pleural cavity of a patient, for subsequent use in placing a larger chest drain;
Figure 9 shows a bronchoscope within the bronchus, with a tissue ablation device in accordance with another embodiment of the present invention being used to ablate a lung tumour proximal to the bronchial wall;
Figures 10 and 11 show the deployed electrodes of tissue ablation devices in accordance with yet other embodiments of the present invention;
Figure 12A shows a tissue ablation device in accordance with another embodiment of the present invention with two deployed electrode coils having opposite polarities;
Figure 12B shows a tissue ablation device in accordance with another embodiment of the present invention with a single deployed electrode coil and where the sheath has an opposite polarity;
Figure 12C shows another embodiment of a tissue ablation device of the present invention configured to ablate tissue in both a bipolar and monopolar manner;
Figure 13 shows a handle coupled to a tissue ablation device in accordance with another embodiment of the present invention; and
Figure 14 shows a flexible deployment handle coupled at an angle to the sheath of a tissue ablation device in accordance with another embodiment of the present invention.

### Description of Embodiments

The invention the subject of this patent application broadly relates to tissue ablation devices intended principally for use in the ablation of tumours in areas of the body that have conventionally been difficult to ablate, and where precision and consistency in ablation is important. For example, ablating lung tumours has conventionally been challenging due to the risk of pneumothorax and because of the lack of uniform conductivity due to the lung's high air content. Other tumours may be located near structures in the body that simply must not be injured, and where positioning of the tissue ablation device and performing the ablation is challenging. The unique structure and functionality of the tissue ablation devices of the present invention makes them useful for ablating such tumours.

As described above, the present invention provides a tissue ablation device. The device comprises a sheath comprising a distal end that is positionable at an ablation site in the tissue, a proximal end and a lumen extending therebetween. The device also comprises one or more electrodes that are advanceable and retractable through the lumen, wherein a distal portion of the one or more electrodes is deployable into a tissue ablating configuration from the distal end of the sheath upon advancement, and the one or more electrodes are removable from the lumen via the proximal end of the sheath upon retraction, wherein the lumen becomes vacated. The sheath is configured for a surgical material to traverse the vacated lumen for delivery from the distal end of the sheath into the tissue.

In the present invention, the tissue to be ablated may be any biological tissue susceptible to thermal coagulation. Typically, the biological tissue required to be ablated will comprise a tumour (usually a tumour which, due to its size, location or other factors, is non-resectable), although benign or malignant soft tissue masses could also be ablated, if clinically required. Tissue which may be ablated in accordance with the present invention includes, for example, pulmonary tissue, liver tissue, brain tissue, as well as the tissue found in a patient's pancreas, thyroid, kidney, uterus or breast.

As would be appreciated, tissue surrounding such tumours may also be ablated in use of the present invention. This may be advantageous because the outer portion of tumours can often be the most malignant, and smaller tumours (which might not yet be detectable) may be spread out from the main tumour mass. Thus, a safety margin of ablation around the tumour may provide for a better oncological outcome. As would be appreciated, however, any ablation around the periphery of a tumour would need to avoid any nearby vital structures.

Although primarily intended for treatment of humans, it is envisaged that the present invention may also be used to ablate tissue in non-human animals.

The device has a sheath which comprises a distal end that is positionable at an ablation site in the tissue, a proximal end and a lumen extending therebetween. The sheath may take any suitable form and may be configured to be positioned in a patient's body tissue using any conventional technique, some examples of which will be described below.

The sheath may be formed from any material compatible with use for its intended purpose. Typically, and especially where the device is adapted for use in percutaneous procedures, the sheath would be formed from metallic materials such as stainless steel or nickel titanium alloys, although plastic materials including Ultem, polycarbonate, polyamide, PTFE and liquid crystal polymer might also be used. Metallic materials may be electrically conductive, which would enable the sheath to act as a return electrode, for the beneficial reasons described below.

In embodiments where the device's sheath is to be inserted into the patient endoscopically (via their airways, for example), the sheath would need to have a high degree of flexibility. Such a sheath may, for example, be formed of polyamide (optionally reinforced with stainless steel braiding), PTFE or other flexible material. Such materials are routinely used in endoscopic devices.

The distal end of the sheath may have a configuration that enables it to penetrate tissue (e.g. like a trocar, for example) or may be non-tissue penetrating. Even if the device of the present invention is indicated for use in percutaneous procedures, to be carried out by interventional radiologists (for example), the distal end of the sheath need not be tissue-penetrating, as it might be inserted using the conventional needle-wire-dilator-sheath technique.

As would be appreciated, it is important to ensure that the tissue ablating device's electrode(s) deploy in the desired manner, regardless of the method used to position the sheath's distal end at the ablation site. One way in which this may be achieved is for the distal end of the sheath to be configured such that it guides an initial direction of deployment of the one or more electrodes. In this regard, the inventors have discovered that providing crenulations at the distal end of the sheath can effectively guide the initial direction of deployment of the one or more electrodes. It may also be advantageous to configure an interior of the sheath such that it include grooves, indentations or ribs (provided that such do not affect the functionality of the vacated lumen) which may also help to guide deployment of the electrode(s).

The crenulations may take any form (e.g. square wave, sine wave, sawtooth or triangular) and may be regular or irregular, depending on factors such as the number of electrodes and their shape, as well as the relative sizes of the sheath and electrodes. The crenulations may also be sharpened to enhance the tissue penetrating ability of the sheath, where appropriate. In some embodiments (e.g. where the sheath is configured for endoscopic insertion), the crenulations may be sharpened on the inside so as to not risk damaging the endoscope.

The proximal end of the sheath may take any form that provides access to the lumen. In the simplest of embodiments, the proximal end of the sheath may comprise an aperture defining a proximal end of the lumen, and into which may be inserted the electrode(s) etc. In other embodiments, however, the proximal end of the sheath may be configured to improve the handleability of the sheath/device and to provide for user-friendly and beneficial interactions with other components of the device. The proximal end of the sheath may, for example, include a guide portion for facilitating easier access into the vacated lumen.

Provided the sheath's distal end can be positioned at the ablation site, any suitable technique may be used to achieve this. In some embodiments, for example, the sheath may be configured for percutaneous (or laparoscopic) insertion, where the ablation device is inserted into the patient through their skin and directly into the body tissue to be treated. In alternative embodiments, for example, the sheath may be configured for endoscopic insertion, where the ablation device is inserted into the patient via an endoscope, and then guided to the ablation site for performing the ablation. Endoscopic techniques that may, for example, be used include bronchoscopy, endoscopy, ECRP (endoscopic retrograde cholangio-pancreatography, a diagnostic procedure conventionally used to examine diseases of the liver, bile ducts and pancreas), cystoscopy and hysteroscopy.

One technique that is routinely used by interventional radiologists in percutaneous insertion procedures, and which may be useful for some embodiments of the present invention, is the so-called "needle-wire-dilator-sheath" procedure. In alternative embodiments (especially when the ablation device is relatively small, as is advantageously possible for many embodiments of the present invention), the sheath may itself be used to penetrate the tissue. The sheath may, for example, incorporate a sharpened end or a crenulated trefoil end, which is sharpened on the inner or outer aspect (or both), and which not only penetrates tissue easily but also aids in the correct deployment of the electrodes (as described herein). In percutaneous procedures, the needle or sheath is carefully inserted through the patient's skin and advanced into a location relative to the ablation site (e.g. a tumour to be treated). Visualisation techniques (e.g. ultrasound (for organs other than the lungs), computerised tomography ("CT") or fluoroscopy), for example, could be employed in order to appropriately position the needle or sheath (i.e. without risk of affecting any endangered atomic structures such as blood vessels, nerves, adjacent organs, etc.). As the needle/sheath have a fine gauge and are hence relatively easy to control during insertion, it is less likely that the operator might accidentally misposition them, with the attendant consequences.

When ablating a lung tumour, for example, a suitable positioning of the patient according to an optimal skin entry site may be determined using CT guidance, allowing the shortest and safest path to reach the target lesion without affecting endangered anatomic structures such as blood vessels or bronchial tubes.

Once so positioned, the sheath remains in the same location throughout the entirety of the ablation procedure. As would be appreciated, this is a much simpler and safer procedure than many conventional ablation techniques, which can require multiple injections.

As noted above, in alternative embodiments, the sheath may be endoscopically positioned at the ablation site in the tissue via one of the patient's body's lumens. The sheath may, for example, be configured for insertion into the patient via their airways, gastrointestinal tract, biliary tree or uterus using suitable image guidance techniques. In one embodiment, for example, the sheath may be carried by a bronchoscope which has been inserted into the patient's airway to a location proximal to a lung tumour requiring ablation (e.g. a lung tumour that is located in close proximity to the bronchial wall). Bronchoscopes having integral visualisation means can be used, for example, to aid in the correct positioning of the sheath with respect to the tumour (i.e. once carried to an area close to the tumour by the bronchoscope).

Endoscopic insertions of the sheath may be useful in situations where tumours may be more easily or safely accessed than via percutaneous insertion. For example, small tumours close to a bronchial wall may be accessed via a sheath that is deployed via a bronchoscope, thereby avoiding the need to puncture the pleural cavity and significantly reduce the risk of pneumothorax. Such an approach would also limit the length of the transpulmonary track and offer increased safety regarding large pulmonary blood vessels, other bronchial tubes and/or adjacent organs. Guidance techniques using real time CT (e.g. Siemens Zeego) may be used to help guide the sheath to the ablation site and the subsequent electrode deployment.

The tissue ablating device of the present invention also includes one or more electrodes that are advanceable and retractable through the lumen. A distal portion of the electrode(s) is deployable into a tissue ablating configuration from the distal end of the sheath upon advancement, and the electrode(s) are completely removable from the lumen via the proximal end of the sheath upon retraction such that the lumen becomes vacated and available for use in the beneficial manner described herein.

Given that one of the applications of the ablation device is for treating tumours in conventionally challenging locations in a patient's body, the electrode(s) would often be deployed into the ablation site (e.g. tumour) itself, there being very little available space to do otherwise. In some embodiments, however, it may be sufficient to deploy the electrode(s) in close proximity to the ablation site/tumour. For example, electrode(s) that assume a helical or circular ablating configuration upon deployment may encompass the ablation site/tumour from an eccentrically placed sheath. This scenario may be necessary, for example, where a tumour is in a very risky location, e.g. on a major blood vessel or close to an important body structure. The sheath itself would not usually be positioned within the ablation site (e.g. inside the tumour), but it would usually be positioned close to the ablation site such that the electrodes deploy into the ablation site in use.

Where the device includes more than one electrode, each electrode may be the same or different to the other electrode(s), and may be configured to deploy in the same or different manner to the other electrode(s). In some embodiments, for example, advantages may be gained by using electrodes which are formed from different materials, which deploy into differently shaped predetermined ablating configurations or which have different deployment lengths.

The electrode(s) deliver RF energy to the tissue into which it is (they are) deployed, and may have any configuration that is compatible with this functionality and not incompatible with other components of the device. The electrode(s) may take many different configurations, with its deployment length and cross-sectional shape and diameter being likely to affect the physical properties of the electrodes, as well as their energy delivery (current, impedance, etc.) characteristics to tissue at the ablation site. The use of electrodes having different diameters and cross sectional shapes may, for example, enable the energy/energy density and distribution in the target tissue to be controlled even more precisely, as well as causing the electrodes to assume different and beneficial predetermined shapes once deployed.

Some cross-sectional shapes of electrodes may also enable a reduction in sheath size (e.g. electrodes having a triangular cross sectional shape instead of a flat shape may be houseable in sheathes that are significantly smaller). Longer lengths of electrode deployed into the tissue would enable more current to be applied, potentially resulting in larger and/or faster ablations, but this must not be at the risk of the electrode being capable of bending back onto itself and potentially causing a short circuit.

The diameter of each electrode may be any diameter appropriate for use in the ablation devices described herein. In some embodiments, for example, an outside diameter of the electrode may be in the range of about 0.1mm to about 2.5mm (e.g. between approximately 0.2mm and about 0.6mm), but is not so limited. Typically, an end of the electrode is adapted for piercing body tissue (i.e. during its deployment), for example by being sharpened.

The cross-sectional shape of the electrode may be any shape suitable for use with the tissue ablating devices described herein. In some embodiments, for example, the electrode or each electrode may have a cross-sectional shape that is independently selected from: circular, a circular segment, elliptical, triangular and flat. As will be appreciated, electrodes having such cross sectional shapes may more reliably deploy into their predetermined ablation configuration and/or produce different energy profiles when ablating selected tissue types. It is within the ability of a person skilled in the art, based on the teachings contained herein, to determine an appropriate electrode configuration for use in the device of the present invention for any given ablation procedure.

The tissue ablating configuration which the deployed electrode assumes may be any suitable shape or configuration that can produce the ablations described herein. Typically, the electrodes would be formed from a material that can be straightened (i.e. to be slidably received in the sheath's lumen) but which is configured to assume a pre-formed shape in the absence of any restraint (e.g. whilst constrained in the sheath's lumen). In some embodiments, for example, the electrode(s) may be configured to recover a pre-formed shape and effectively bend into a coil in the body tissue upon deployment, this being something readily achievable using conventional electrode materials.

In such embodiments, the deployed configuration of the electrode(s) may be helical in shape, which the inventors have found can result in relatively longs amount of the electrode(s) being deployable into the ablation site, which may enable more energy to be delivered and hence an even more effective ablation. The helical electrode deployment shape described in further detail below has, in particular, been found to have significantly more electrode deployed, when compared to more conventional circular deployments. The helical electrode can also coil upon itself by over 360°, and deploys outwardly from the sheath's distal end, which the inventors have found to result in particularly beneficial ablations, as well as a reduced risk of contact with the sheath (which might cause a short circuit in some embodiments). The helical shape of the deployed electrodes have been observed by the inventors (using CT and ultrasound imaging) to be maintained in tissue.

Such helical electrodes may deploy clockwise or counter clockwise, and circular helical electrodes would have a constant radius and curvature. Also envisaged, however, are electrodes which deploy into a conic helix, a double helix or a slant helix, as these may provide for beneficial ablation configurations for given tumours or tissue.

The deployed configuration(s) of the electrode(s) may have any suitable size, bearing in mind the overarching requirement that the device is intended for ablating tumours in conventionally challenging locations, where precision and consistency in ablation is paramount. Fewer electrodes and/or smaller electrodes (i.e. having a smaller calibre, coil or length) are thus generally preferred. In some embodiments, for example, the deployed configuration of a generally-circularly-shaped electrode may have a diameter of 0.5cm or less. The inventors expect that ablations of up to about 2-4cm would be achievable using such electrodes in the ablation devices of the present invention.

The electrode(s) may be used to create an ablation having an appropriate volume. In some embodiments, for example, ablation volumes having a diameter in the range of about 1-2cm are envisaged in organs such as the lungs or uterus. Larger ablations (up to about 6cm, for example) may however be performed in organs such as the liver (e.g. using the multiple ablation procedure described herein).

The electrodes may be formed from any electrically-conductive material, although they may also include non-conducting materials, coatings, and/or coverings in various segments and/or proportions (e.g. for insulation purposes), provided that such are compatible with the deployment and energy delivery requirements of the corresponding procedure and/or the type of target tissue.

As noted above, in some embodiments, the electrode(s) may be configured to recover a pre-formed shape. In such embodiments, the electrode(s) may include or be formed from materials that support bending and/or shaping of the electrodes post-deployment. The electrodes may, for example, include pre-bent wire which, once deployed from the confines of the sheath's lumen, is free to assume its bent configuration. Examples of materials which may be used to form the electrodes of the present invention include stainless steel, carbon steel, B-Titanium or nickel-titanium alloys, such as those sold as "Nitinol Wire" by Fort Wayne Metals.

Nitinol Wire, for example, has excellent biocompatibility and hyper elastic properties which have been found to be useful for coiled/helical electrodes. Nitinol can undergo greater mechanical deformation than other suitable electrode materials before becoming permanently deformed (i.e. it is "non-kinking"), which is beneficial because the electrodes undergo significant deformation when they are retracted within the sheath. Electrode coils made from Nitinol have been found to have tighter radiuses than alternative metals of the same diameter stock, and to result in stronger devices having reliable electrode deployment and retraction. The non-kinking properties of Nitinol Wire would also be advantageous because it might not be possible to retract a kinked electrode back into the sheath.

Stainless steel electrodes have also been found to yield good ablations and perform well in regards to deployment and penetration.

Another complication when ablating tumours in many of the conventionally challenging locations described herein is the need for the ablation device to be relatively small. However, smaller ablation devices have attendant problems, which include the risk of the correspondingly relatively small electrodes breaking and/or not being capable of delivering sufficient energy to cause an effective ablation. The inventors have identified these problems and devised unique solutions, often going against conventional wisdom in doing so.

The inventors recognised, for example, that significant and unexpected advantages could be gained by configuring the electrode(s) for single use only. The electrodes of conventional ablation devices (especially the larger devices, e.g. those for ablating relatively large tumours in organs such as the liver) are integral to the devices and indicated for reuse multiple times. In light of the present invention, however, the ability to remove the electrode(s) easily facilitates their disposal after they have been used, with fresh replacement electrodes being introducible into the vacant lumen as necessary. This advantageously avoids the need to reuse electrodes, which may be compromised (even if they do not visibly appear to be so) and thus not perform adequately or which might fail (either structurally or electrically) during use.

Such embodiments of the invention have been found to provide numerous advantages, including effectively eliminating the risk that cumulative ablations will erode the electrode and increase the risk of it breaking during each successive deployment or retraction. A new electrode, and one which is highly unlikely to erode to an extent where it might break during ablation (retraction of the electrodes back into the sheath post-ablation is likely to be the time when electrodes would break), may instead be used for each ablation. Furthermore, materials which may not otherwise be used due to durability concerns, but which provide beneficial properties, may be used.

For example, in embodiments of the present invention, the one or more electrodes may be formed from carbon steel, which is a more efficient conductor and which more reliably deploys than electrodes formed from the conventionally-used stainless steel. Carbon steel tends not to be used as electrodes in conventional ablation devices, however, because it is difficult to clean and tends to become tarnished.

Similarly, the 'shape memory' properties of Nitinol make it unsuitable for multiple uses. Multiple uses of Nitinol electrodes results in a progressive change in the electrode's shape, which is not consistent with the precise electrode positioning requirements during ablations. The present invention enables the beneficial properties of such materials to be utilised, but without the attendant disadvantages associated with multiple uses of those materials. Furthermore, single use electrodes can simply be disposed and thus do not require cleaning, and their use therefore reduces the risk of stick injuries which may otherwise occur during cleaning.

The electrode(s) may be advanced and retracted through the sheath's lumen in any suitable manner, for example as will be described in further detail below.

In some embodiments, the device's one or more electrodes may comprise a single electrode which assumes its ablating configuration post-deployment. In other embodiments, however, the one or more electrodes may comprise a plurality of electrodes (e.g. 2 or 3 or more electrodes), each of which assumes the same or a different ablating configuration (e.g. relatively larger or smaller and/or having a different shape) upon deployment. In such embodiments, an electrode ablating configuration may be provided that gives a functionality not achievable by a single electrode. Such embodiments may be beneficial in ablating relatively larger or uneven shaped tumours, for example, where electrodes having a composite shape are better able to ablate the tumour (e.g. because of the shape of the composite deployed electrodes and/or intensity of the RF energy applied by the electrodes).

Each of the electrodes may be configured to be deployed independently of or concurrently with the other electrode(s). Each of the electrodes may be deployable through a respective orifice at the end of and/or along a side of the distal end of the sheath.

The electrodes in such embodiments of the present invention may be electrically connected to or insulated from each other, and may have the same or a different polarity to each other. The number of electrodes in such embodiments is limited only by the physical constraints and functional requirements of the ablating device of the present invention.

In some embodiments, the area immediately proximal to the deployable distal end of the electrodes may be joined in order to prevent rotation of the electrodes within the sheath, which might otherwise be experienced as the electrodes are penetrating certain tumour types. Such joining may be effected by soldering, welding, banding, braiding or with a suitable adhesive.

Electrically insulating materials would be used in the ablation device as necessary. For example, where there are two or more electrodes, these may need to be electrically insulated from each other whilst in the sheath's lumen, and the portion of the electrodes which remain in the sheath post deployment will need to be permanently insulated. In some embodiments, the sheath, or a portion thereof (i.e. which is positioned in the tissue during ablation), may also be electrically active (e.g. itself providing a return electrode), and insulation would therefore also be required between the sheath and the electrodes.

As will be appreciated, smaller electrodes might be severely damaged in the event of a short circuit. Safeguards (either physical or electrical) may therefore also be provided that prevent electrical current from being applied until such time as the sheath has been positioned at the ablation site and/or the electrodes deployed into their ablating configuration. Safeguards may also be provided that prevent electrical current from being applied if there is any risk of an electrical short circuit occurring. The device might also employ an automated deployment mechanism, which ensures that complete deployment has occurred before ablation can begin.

In some embodiments, insulating materials used in the device may be lubricious. Lubricious insulating materials can improve the ability of the electrode(s) to move relative to each other and the sheath. Any suitable insulating material may be used to overlay at least a portion of the one or more electrodes. In some configurations, for example, the insulating material may comprise a polymeric material (e.g. PTFE, fluorinated ethylene propylene, high density polyethylene, polyethylene).

As will be described in further detail below, some embodiments of the present invention provide for a "hybrid monopolar and bipolar" ablation method and system, where the ablation device is operable such that the characteristics of ablations conventionally achieved using both bipolar and monopolar devices are achieved. In such embodiments, the electrode(s) may be configured to have a first polarity during ablation, whereby ablation occurs between the electrode(s) and a grounding pad having a second (i.e. opposite) polarity positioned on the body of a patient. The electrode(s) may also (or instead) be configured to have a polarity during ablation opposite that of an electrically conductive portion of the sheath, whereby ablation occurs between the electrode(s) and the electrically conductive portion of the sheath. The one or more electrodes may also (or instead) comprise a plurality of electrodes configured to have opposite polarities during ablation.

As will be described below, this unique combination of polarity configurations may enable the devices of the present invention to produce ablations having a precision and consistency not previously obtainable, even in body tissue which has conventionally been challenging to ablate. Indeed, in some embodiments of the present invention, the inventors have found that the ablations which can produced by devices having a given sheath diameter are significantly larger and more precise than those achievable using similarly sized conventional devices. The benefits of smaller diameter sheaths are relevant to all tumours (i.e. not just lung tumours, where the sheath diameter is directly related to the risk of pneumothorax), with less trauma generally being associated with smaller sheathes. The unique configuration of the devices of the present invention, and especially when used in the hybrid mono-bipolar ablation system of the present invention, can therefore provide significant advances over conventional ablation devices.

In some embodiments, where the tissue ablating configuration of the deployed electrode(s) is substantially planar (i.e. this embodiment is less relevant for the helical tissue ablating configuration described herein), the one or more electrodes may be deployable from the distal end of the sheath with an angle of deployment that is selectable by orientating the electrodes with respect to the sheath. As described in detail in co-pending PCT application no. PCT/AU2019/050880, entitled DEVICES AND METHODS FOR ABLATING BIOLOGICAL TISSUE, such a feature enables smaller devices having smaller electrodes to be used to ablate relatively large volumes of tissue.

In some embodiments, the proximal end of the sheath may comprise means for indicating a relative orientation between the angle of deployment of the (planar) electrode(s) and the sheath. Such means may help an operator to ensure that a desired ablation pattern is achieved, notwithstanding them possibly not being able to physically see (i.e. using imaging techniques) the deployed electrodes. The proximal end of the sheath may, for example, comprise visual or tactile means for indicating a relative alignment therebetween.

The various embodiments of the present invention described above provide the operator with an unprecedented degree of versatility in performing ablations, with a variety of electrodes being deployable through the lumen of the pre-placed sheath at a variety angles into the tissue surrounding a tumour.

The electrode(s) of the devices of the present invention need to be electrically connected to an energy source in order for ablation to occur. Such an energy source may be provided in the form of an electrical generator which can deliver pre-specified amounts of energy at selectable frequencies in order to ablate tissue. The energy source may include at least one of a variety of energy sources, including electrical generators operating within the radio frequency (RF) range. More specifically and by way of example only, the energy source may include a RF generator operating in a frequency range of approximately 375 to 650 kHz (e.g. 400 kHz to 550 kHz) and at a current of approximately 0.1 to 5 Amps (e.g. of approximately 0.5 to 4 Amps) and an impedance of approximately 5 to 100 ohms. As would be appreciated, variations in the choice of electrical output parameters from the energy source to monitor or control the tissue ablation process may vary widely depending on tissue type, operator experience, technique, and/or preference.

In the tissue ablation device of the present invention, the lumen becomes vacated upon complete withdrawal of the electrode(s) therefrom, with the sheath thus being configured to receive surgical material for delivery into the tissue therethrough. The vacant lumen advantageously provides a conduit for the surgical material directly into the ablation site, as well as the track via which the sheath reached the ablation site.

Any surgical material that may have beneficial effect and that is compatible with insertion via the sheath's lumen may be used. Flowable surgical materials can readily be delivered into and through the vacant lumen using conventional techniques, some of which are described herein. Non-flowable surgical materials of appropriate size may also be delivered into and through the vacant lumen using conventional techniques.

The vacant lumen may also or instead provide access to the ablation site/track for a first surgical material (e.g. a guide wire), which facilitates the insertion of a second surgical material (or device) into the track (e.g. either before or after the device's sheath is withdrawn from the patient's body).

In some embodiments, for example, a surgical material in the form of a tissue adhesive may be delivered via the vacant lumen into the patient whilst the sheath is initially withdrawn out of the patient (i.e. where it seals any hole between the lungs and pleural cavity),

In some embodiments, for example, the vacant lumen may be used to place a surgical material (e.g. a drain, such as a pleural drain) in an appropriate position within the patient, the sheath being completely withdrawn out of the patient once the surgical material is in position. In such embodiments, the surgical material may be placed after the sheath has been partially withdrawn from the patient (e.g. when the sheath's distal end is located in the pleural cavity).

In some embodiments, for example, a surgical material in the form of a tissue adhesive may be dispensed via the vacant lumen into the patient whilst the sheath is initially withdrawn out of the patient (i.e. where it seals any hole between the lungs and pleural cavity), with the drain subsequently being placed (e.g. in the pleural cavity).

The tissue ablation device may optionally also include a dispenser that is operable to dispense the surgical material into the vacant lumen. Any dispenser that is capable of storing (even if only temporarily) the surgical material and which is capable of causing it to be inserted into and through the sheath's vacant lumen may be used in the present invention.

Typically, the dispenser would be configured for attachment to the proximal end of the sheath. The dispenser may, for example, have a dispensing portion that is configured to couple to an appropriately configured portion at the proximal end of the sheath. In some embodiments, for example, the dispenser may be provided in the form of a syringe having a nozzle that is coupleable to the sheath's proximal end (or a housing provided thereat), a barrel for storing a flowable surgical material and a plunger that is manually operable by the user to dispense the flowable substance as required and in a conventional manner.

The tissue ablation device of the present invention may include additional features or components, where these may provide advantages in terms of its utility or handling. The device may for example, in some embodiments, include a handle in order to improve its operability.

In some embodiments, for example, the ablation device may further comprise a deployment handle that is operable to advance and retract the one or more electrodes through the lumen. Such a handle may help the operator to deploy the electrodes with a greater degree of control than they may otherwise be able to achieve. Such a handle may be ergonomically configured to enable an operator to manipulate the device in the required manner, and may also be used to help insert the sheath into the tissue.

The deployment handle may, for example, be removably attachable to the device. Such a handle can therefore be removed from the device with the electrode(s), for example, in order to provide or enhance access to the sheath's proximal end and hence the vacated lumen.

In some embodiments, the deployment handle may be configured such that the physical constraints imposed during the ablation of some kinds of tumours can be anticipated. For example, ultrasound techniques are not generally able to be used to visualise lung tumours, and cannot therefore be used to help appropriately position the device's sheath. Instead, visualisation techniques such as CT visualisation must be used, which severely restricts the space available for the surgeon or interventional radiologist to work.

In such circumstances, therefore, the deployment handle may be orientated at an angle to the sheath. In some embodiments, the deployment handle may instead or also comprise a flexible portion. These features can help to shorten the overall length of the ablation device (especially when the handle is pulled back, i.e. before the electrodes have been deployed), and thereby give the operator a better clearance for them to work. Use of the highly flexible and resilient Nitinol Wire or carbon steel electrodes may be advantageous in such embodiments.

In some embodiments, the handle may itself include safety features. For example, the handle may be configured to prevent commencement of ablation until the electrodes have been completely deployed.

In some embodiments, the tissue ablation device may also include an impedance detector attached to one or more of the sheath and electrode(s). The impedance detector may be configured to detect the impedance of the surrounding tissue, which data can be used to control the rate or progression of the ablation.

The tissue ablation device may also include a temperature sensor configured to detect the temperature of the surrounding tissue. The temperature sensor may be attached or integrated to one or more of the sheath and electrode(s), and provide data which can be used to control the rate or progression of the ablation.

Some embodiments may further include a feedback mechanism configured to change the amount of power applied to the electrode(s) (etc.) and hence tissue at the ablation site in response to one or more monitored attributes. The one or more monitored attributes may, for example, be selected from the group consisting of tissue temperature, tissue impedance and ablation time. In some embodiments, for example, the feedback mechanism may be configured to stop or adjust the application of power applied to the tissue in response to a level of the one or more monitored attributes exceeding a predetermined limit.

The invention also provides methods for ablating tissue at an ablation site in a patient's body. In one form, the method comprises:
providing a device for ablating tissue, the device comprising:
   a sheath comprising a distal end that is positionable at an ablation site in the tissue, a proximal end and a lumen extending therebetween;
   one or more electrodes that are advanceable and retractable through the lumen, wherein a distal portion of the one or more electrodes is deployable into a tissue ablating configuration from the distal end of the sheath upon advancement, and the one or more electrodes are removable from the lumen via the proximal end of the sheath upon retraction, whereupon the lumen becomes vacated; and
   wherein the sheath is configured to receive a surgical material for delivery into the tissue via the vacated lumen;
positioning the distal end of the sheath in the patient at the ablation site;
deploying the one or more electrodes into the tissue ablating configuration and ablating tissue;
retracting the one or more electrodes back into the sheath and subsequently removing the one or more electrodes from the lumen; and
delivering the surgical material into the patient via the vacated lumen.

The inventors recognised that providing a conduit into the ablation site and the track via which the sheath reached the ablation site would provide a number of significant advantages, perhaps the most significant of which is that the entire procedure can potentially be carried out using only one (relatively small) incision. The benefits of this will be readily apparent to a person skilled in the art, and include less trauma to the patient (and hence a faster recovery), a simplified procedure (able to be carried out by interventional radiologists instead of surgeons), a reduced risk of post-procedure bleeding and, in the case of lung ablations, a significantly reduced risk of pneumothorax.

In some embodiments, the surgical material may be a flowable surgical material that is dispensed into the proximal end of the sheath and which flows through the vacated lumen, out f the distal end and into the patient. In some embodiments, for example, a dispenser containing a flowable substance may be coupled to the proximal end of the sheath and operated to dispense the substance via the vacated lumen into the patient.

In other embodiments, the surgical material may be a non-flowable surgical material which is dispensed into the proximal end of the sheath and pushed through the vacated lumen and into the patient. Any suitable mechanism for advancing such a surgical material through the lumen may be used. Typically, the surgical material would be physically pushed through the vacated lumen, for example using a plunger or the like.

In some embodiments, where it might be advantageous to do so ,two (or more) surgical materials (flowable and/or non-flowable) may be delivered into the patient via the vacated lumen. For example, a track-sealing or occluding device (removable or implantable) may be deployed through the vacated lumen of the sheath after removal of the electrodes. It is envisaged that this would be used in addition to a flowable substance such as tissue glue to provide an immediate tamponading effect to prevent expulsion of the flowable substance. It has been discovered by the inventors that this tamponade can significantly improve the sealing effect of such flowable substances.

The surgical material may be delivered at any time during the procedure, with the timing being determined based on the intended effect of the substance. In some embodiments, for example, the surgical material may be delivered whilst the sheath is being withdrawn out of the patient. Delivering surgical materials such as tissue glue whilst the sheath is being withdrawn out of the patient would result in the tissue glue sealing the track of the sheath, which may help to prevent bleeding and (in embodiments where a lung tumour was being ablated) seal the hole between the lung and the pleural cavity. Dispensing tissue glue (for example) into the sheath's proximal end whilst the device/sheath was being withdrawn from the ablation site to the pleural cavity may result in a volume of tissue sealant being delivered which is not easily expulsed by air pressure (i.e. before it can set).

In some embodiments, it may be advantageous to deliver a surgical material before performing the ablation.

Flowable surgical materials which might be delivered at an appropriate time include a tissue glue, a chemotherapeutic agent (e.g. to kill any tumour cells that may have survived the ablation procedure and prevent them seeding along the track), a pro-coagulant (i.e. to stop bleeding even more quickly). Combinations of flowable substances (delivered separately or in combination, depending on the substances) may also be used if there was an advantage in doing so.

Non-flowable surgical materials which might be dispensed at an appropriate time include pleural drains, plugs, occluding devices or guide wires (that can subsequently be used to guide other items into the track using conventional techniques).

The method of the present invention requires the distal end of the sheath to be positioned in the patient at the ablation site. As described above, typically, the distal end of the sheath would be positioned as close to the tumour (ablation site) as possible, but not usually inside it. That being said, however, in some embodiments, and especially those where the distal end of the sheath (or a portion of the sheath close to its distal end) acts as a return electrode, the heat generated at the sheath may advantageously contribute to the ablation.

The distal end of the sheath may be positioned in the patient at the ablation site using any suitable technique. As a person skilled in the art would appreciate, two potentially suitable techniques include percutaneously inserting the sheath through the patient's skin and endoscopically inserting the sheath via any lumen in the patient that is accessible endoscopically. For example, the sheath may be configured to be positioned at the ablation site via the patient's airway (i.e. when treating lung tumours) using a bronchoscope. Alternatively, the sheath may be configured to be positioned at the ablation site in a patient's gastrointestinal tract, uterus, kidneys, bladder, liver, bile ducts and pancreas using an appropriate endoscope. Examples of such techniques are known in the art and, based on the teachings contained herein, could be adapted for use in the methods of the present invention.

Endoscopes used to carry the tissue ablating device's sheath through the respective body lumen would generally only be configured to carry the sheath along the patient's lumen and would not generally be configured to penetrate tissue in order to reach the ablation site. In such embodiments, therefore, the sheath may be configured to be deployable from the distal end of the endoscope at an appropriate time, where it is manoeuvrable into an appropriate position for ablation. The sheath itself may be used to penetrate the lumen (e.g. bronchial wall), if such is necessary in order to position its distal end at the ablation site. Alternatively, positioning the distal end of the sheath against the bronchial wall (for example) may suffice, with the electrodes piercing the bronchial wall when being deployed. Techniques and endoscopic devices for performing such manoeuvres are known in the art.

Endoscopic insertion might be chosen instead of percutaneous insertion if the tumour was readily accessible using the endoscope, or if the ablation site is located relative to important structures such that percutaneous insertion would be too risky. In such cases, endoscopic insertion may effectively provide a "different angle" from which to access a tumour.

In embodiments of the invention, the ablation site may comprise a tumour in the lung, pancreas, liver, thyroid, kidney, uterus, brain or breast of the patient. Fibroids or soft tissue lesions may also be ablated using the methods of the present invention.

In a more specific form, the method of the present invention is used to ablate lung tumours in a patient. In such a form, the method comprises:
providing a device for ablating lung tumours, the device comprising:
   a sheath comprising a distal end that is positionable at the lung tumour, a proximal end and a lumen extending therebetween;
   one or more electrodes that are advanceable and retractable through the lumen, wherein a distal portion of the one or more electrodes is deployable into a tissue ablating configuration from the distal end of the sheath upon advancement, and the one or more electrodes are removable from the lumen via the proximal end of the sheath upon retraction, whereupon the lumen becomes vacated; and
   wherein the sheath is configured to receive a surgical material for delivery into the tissue via the vacated lumen;
percutaneously positioning the distal end of the sheath at the lung tumour;
deploying the one or more electrodes into the tissue ablating configuration and ablating tissue including the lung tumour;
retracting the one or more electrodes back into the sheath and subsequently removing the one or more electrodes from the lumen; and
dispensing tissue glue (or, in other forms, an occluding device or an occluding device in conjunction with a tissue glue) into and through the vacated lumen as the sheath is partially withdrawn out of the patient, whereby a hole between the patient's lungs and their pleural cavity is sealed.

As noted above, ablation of tumours in the lungs can be particularly challenging. Air is inherently a relatively poor conductor of heat and electricity, and ablations in pulmonary tissue may not progress in the same manner as would, for example, occur in more dense tissue (e.g. in a liver). Blood vessels, the close proximity of vital organs such as the heart and the ever-present risk of pneumothorax all add to the complexity associated with the ablation of lung tumours. As also noted above, visualisation of the tumour and the ablation device can also necessitate the use of CT instruments, with the attendant physical space constraints and hence complexities to the procedure.

Ablations in other locations in the body can also be complicated by factors such as the proximity of important structures and access pathway to the ablation site. Advantageously, the present invention provides a device and method which can, for the reasons discussed above, minimise the risk of complications occurring during such procedures.

The present inventors have also discovered a unique ablation method, which is a hybrid of the conventional monopolar and bipolar ablation techniques. The inventors' preliminary experiments indicate that their novel method will enable relatively small ablation devices to produce larger and more controlled ablations, even in organs such as the lungs where ablation is complicated by the presence of non-conductive air. The present invention therefore also provides a method for ablating tissue at an ablation site in a patient's body. The method comprises:
providing a device for ablating tissue, the device comprising:
   a sheath comprising a distal end that is positionable at an ablation site in the tissue, a proximal end and a lumen extending therebetween; and
   one or more electrodes that are advanceable and retractable through the lumen, wherein a distal portion of the one or more electrodes is deployable into a tissue ablating configuration from the distal end of the sheath upon advancement;
positioning the distal end of the sheath in the patient at the ablation site, and a grounding pad on the patient's body;
deploying the one or more electrodes into the tissue ablating configuration in the ablation site; and
causing ablation to occur wherein, during ablation:
   at least one of the one or more electrodes is caused to have a polarity that is opposite to that of the grounding pad, and
   an electrically conductive portion of the sheath or another of the one or more electrodes is caused to have a polarity that is the same as that of the grounding pad,
   whereby ablation progresses according to a relative impedance of tissue at the ablation site.

The inventors discovered that the synchronous provision of a return path (i.e. from the deployed electrode) through a portion of the ablation device's sheath and a separate patient applied grounding pad (also known as an earth plate) allows balancing of radiofrequency delivery between these return electrodes, until such time as an impedance limit is reached. In effect, ablation occurs between the electrodes and grounding pad at the same time as between the electrodes and the uninsulated portion of the device's sheath, with the impedance of the tissue therebetween governing the relative energy distribution. This process can provide a precise and complete ablation from the deployed electrode(s) to the uninsulated sheath. Progressively the impedance of this circuit rises and more energy is diverted to the grounding pad on the patient, until no electrical circuit exists in either bipolar or monopolar mode. The inventors discovered that the concurrent use of the grounding pad and conductive sheath can result in larger, more controllable and predictable, and more complete ablations than when using the sheath or pad alone as a return electrode.

Furthermore, a more uniform heat distribution over the ablation site can be achieved using the hybrid mono-bipolar method, contributing to a more effective ablation. In contrast, conventional ablation methods often result in effective ablation immediately surrounding the electrode, but one which becomes much less effective with distance and would therefore usually require the use of relatively larger electrodes or multiple insertions in order to ablate a sufficient volume.

The inventors have found that it is not necessary to cause switching between electrodes, as is the case for some prior art devices, with the path of return of radiofrequency energy initially occurring preferentially to the sheath but, as impedance rises due to the coagulation of tissue around the sheath, the path of return of radiofrequency energy progressively passes to the patient grounding pad to return to the generator. This process continues until the "active" electrode is completely impeded out, and can result in ablations having a larger and more precise volume than would otherwise be expected to be achievable using monopolar or bipolar devices (having comparable electrode sizes) in isolation. Furthermore, the inventors expect that the position of the earth plate on the patient relative to the ablation site may be able to cause ablations having a particular shape, which may be advantageous when ablating some tumours.

The size of the ablations carried out using the methods of the present invention are unlikely to be as large as those which can be produced using conventional techniques, where identical probes are positioned on either side of a tumour and their deployed electrodes subsequently used to produce large and predictable ablations therebetween. When treating tumours in the lung, however, the use of devices having a single needle/sheath is favoured because of the risk of air leakage from the lung (the use of two radiofrequency needles would significantly increase this risk). Similarly, the risk of bleeding or injuring an important body structure increases with the number of needles/sheathes inserted, and some tumours may simply not be accessible to two sheathes. Furthermore, and as noted above, the smaller the sheath that is to be inserted into the patient's body, the better. This being said, selection of appropriate electrode materials, deployed ablation configurations and ablation methods in accordance with the teaching of the present invention should enable ablation devices having relatively small sheathes to produce relatively large ablations.

Specific embodiments of the devices and methods of the present invention will now be described with reference to the accompanying drawings. It will be appreciated that the embodiments described below are illustrative in nature and are in no way intended to limit the scope of the present invention. For example, although described below mainly in the context of ablating pulmonary tissue (containing lung tumours), it will be appreciated that the tissue ablating devices, ablation methods and systems of the present invention may also be used to advantage when ablating tumours/tissue in other areas of the body, including those described above.

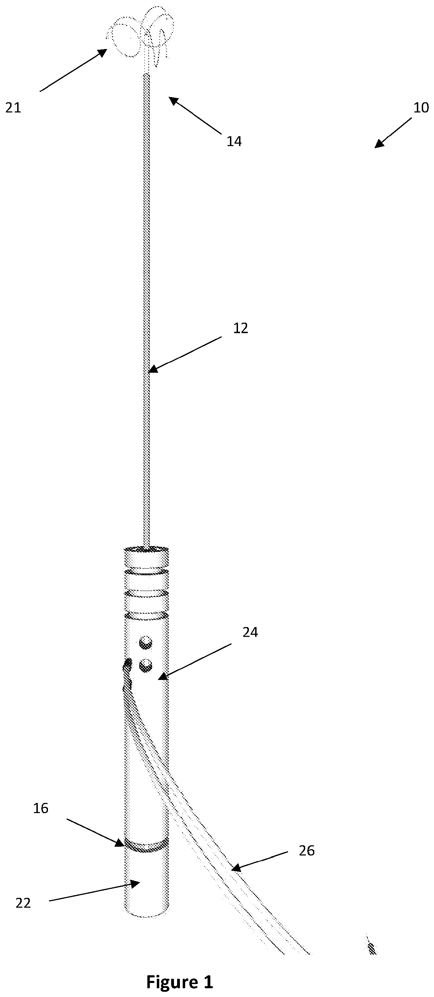

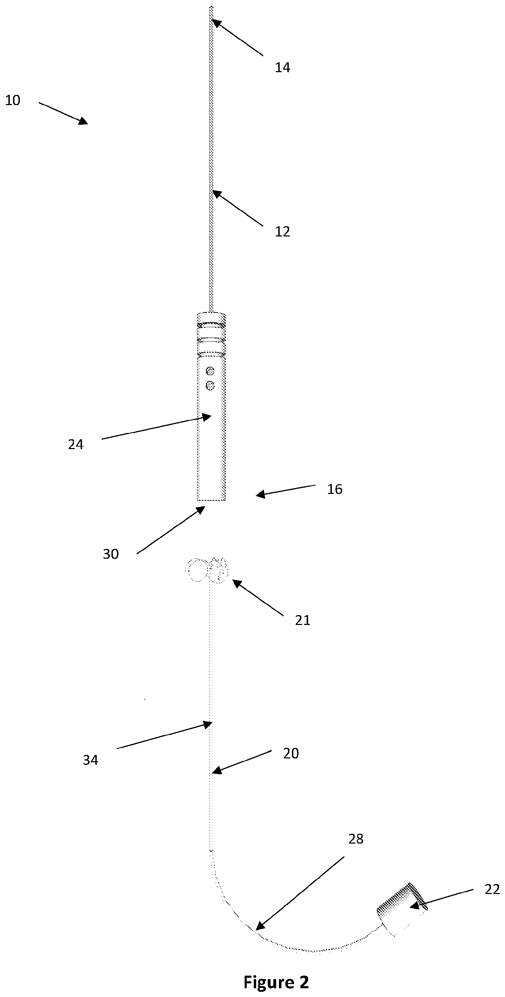

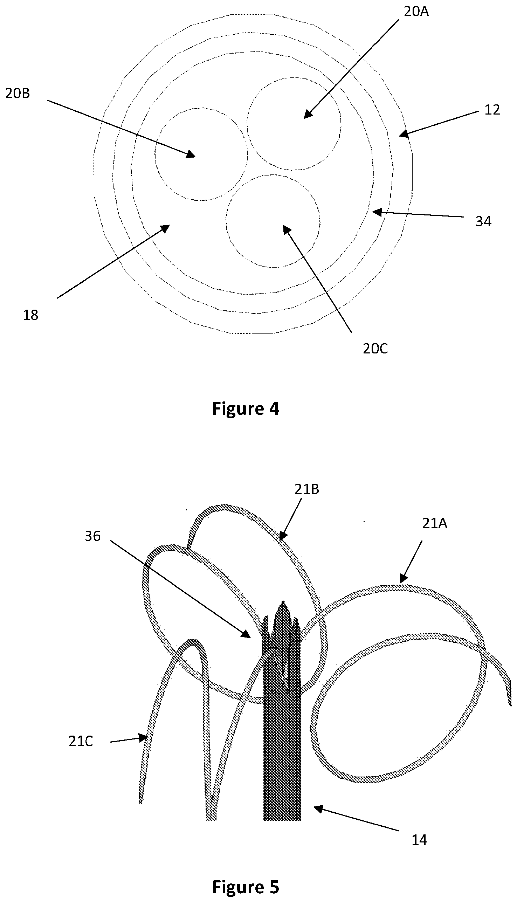

Referring firstly to Figures 1 and 2, shown is a tissue ablation device 10 in accordance with an embodiment of the present invention. The device 10 has a sheath 12 which has a distal end 14 that is positionable (in use, e.g., as described below) at an ablation site in a patient's tissue, a proximal end 16 and a lumen 18 (see Figure 4) extending between the distal 14 and proximal 16 ends. The device 10 also includes three electrodes, shown generally in Figures 1 and 2 as electrodes 20. As shown in Figure 1, electrodes 20 are mostly located within the lumen 18 (where they cannot be seen), with only their distal portions 21 shown in their tissue ablating configurations projecting outwardly from the distal end 14 of the sheath 12. The distal ends 21 of the electrodes 20 are configured to assume the helical conformation shown in the Figures when not subject to any other constraining forces (e.g. as would be experienced in the lumen 18). In the embodiment shown, the electrodes 20 may be formed from Nitinol Wire, the distal ends 21 of which having been configured to assume the predetermined shapes shown in Figure 1 upon deployment in the manner described herein.

Electrodes 20 are advanceable and retractable through the lumen 18 by a relative sliding movement (usually the electrode would be moved whilst the sheath 12 remains stationary), which can be effected by a user pushing and pulling a deployment knob 22. The electrodes 20 are retractable and subsequently removable from the lumen 18 via the sheath's proximal end 16 upon an appropriate retraction of the knob 22, as shown in Figure 2.

A handle 24 may also be provided over the proximal end 16 (or at least a portion thereof) in order to make the sheath 12 easier for the surgeon or interventional radiologist to manipulate. Cables 26 are shown which can provide a source of electrical current to the sheath 12 and/or electrodes 20. In embodiments of the ablation device 10 indicated for the treatment of lung tumours, the diameter of sheath 12 will be approximately 1.6mm.

Knob 22 is joined to the electrodes 20 via a flexible cable 28 (see Figure 2), which enables the overall length of the device 10 to be minimised for use in confined spaces such as the inside of a CT. Pulling knob 22 away from the handle 24 would initially cause the electrodes 20 to be drawn back into the lumen 18, with their helically-shaped distal portions 21 being straightened when doing so. Yet further movement of knob 22 would further retract the electrodes 20 into the sheath 12, until they are eventually completely retracted and subsequently removed from the device 12 via proximal end 16.

Knob 22 is manually actuated but in alternative embodiments (not shown), an automated deployment and retraction mechanism might be used. Such an automated mechanism might be advantageous in that it can provide for reliable, consistent and accurate electrode deployment. Over deployment of the electrodes 20 might cause them to kink and/or make contact with conductive portions of the sheath 12, potentially causing a short circuit. Under deployment might also cause a risk of short circuiting, and may also result in sub-optimal ablations. In alternative embodiments, the knob and/or device may be provided with other means by which the optimal electrode deployment can be assessed by the operator. Such means may, for example, include tactile or audible means (e.g. a "Click" is felt by the operator) or visual means (e.g. a red/green portion of the handle which is indicative of optimal deployment). In some embodiments (again, not shown), the device may include a safety mechanism, whereby electrical current cannot be applied to the device until such time as the proper ablation configuration of the electrodes has been achieved).

As will be appreciated, once the electrodes 20 have been removed (i.e. as shown in Figure 2), unhindered access to the now-vacated lumen 18 is provided via the proximal end 16 of the sheath 12. In the embodiment shown in Figures 1 and 2, a coupling 30 (see Figure 2) is provided which is configured to receive a syringe (not shown) threat. It is therefore a simple matter to connect a syringe containing tissue glue sealant to the sheath 12 via coupling 30, and to inject the sealant through the vacant lumen 18 and out of the distal end (e.g. in order to plug the track in the lung whilst removing this sheath to reduce the risk of pneumothorax, as described above). The lumen 18 (and sheath 12) may subsequently be cleaned (e.g. using a suitable solvent) or th sheath may, in some embodiments, be intended for a single use only.

In alternative embodiments (not shown), other surgical materials such as sealing devices may be inserted into the patient via the vacant lumen 18 to achieve a beneficial effect. In some embodiments (not shown), for example, a balloon catheter may be inserted through the lumen 18 and used in combination with a tissue sealant for tamponading the sealant whilst it cures. This may help to prevent air from pushing the sealant out of a hole in the lung/pleural cavity before it has time to cure.

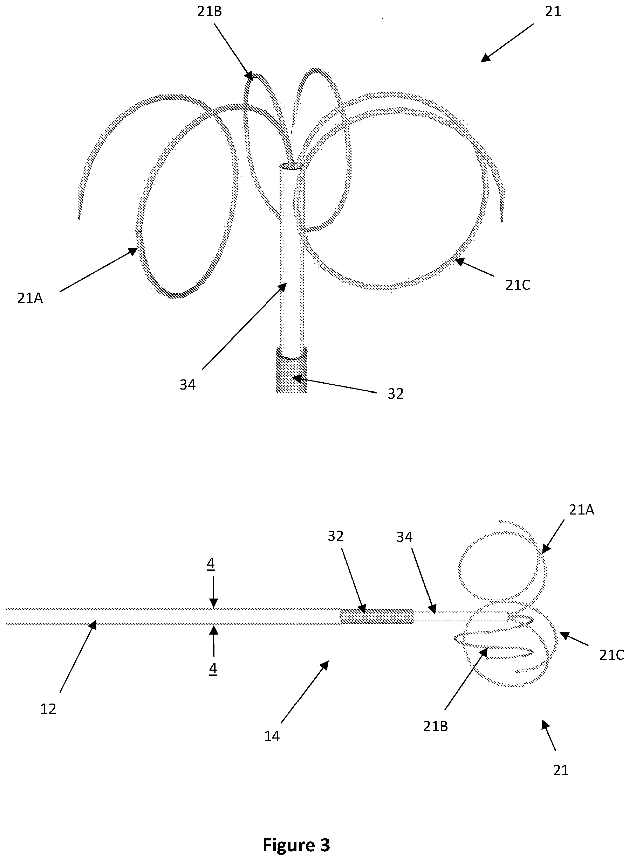

Referring now to Figures 3 and 4, the ablating configurations of the distal ends 21 of the electrodes 20 will now be described in further detail. As noted above, the inventors have discovered that a helical shape of the distal ends 21 of the deployed electrodes 20 enables a relatively long length of the electrodes to be deployed, with there being little risk of any of the electrodes returning to the sheath or towards each other and potentially causing a short circuit. As would be appreciated, circular electrode deployment configurations result in the distal end (leading edge) of the electrode returning towards the distal end of the sheath, which limits the amount of electrode that can be deployed. The inventors note that the amount of energy that can be delivered to an ablation site is, generally speaking, proportional to the length of the electrode in the tissue. Thus, generally speaking, electrodes which can be safely deployed into configurations where a relatively long length of the electrode is deployed into the tissue should be more effective in achieving larger ablation volumes and faster, whilst not necessarily increasing the diameter of the needle track that is created as the device is inserted.

The distal portions 21A, 21B and 21C of the three electrodes 20A, 20B and 20C respectively can be seen projecting out of the distal end 14 of sheath 12. Each electrode terminates at a sharpened end (not numbered) in order for it to readily penetrate tissue (noting that tumour tissue can often be relatively hard and/or resilient). The distal end 14 of sheath 12 includes an uninsulated portion 32 which, when appropriately connected to a source of electricity, can act as a return electrode for the beneficial ablative effects described below. In such embodiments, it is essential that the electrodes 20 cannot make electrical contact with uninsulated portion 32, and an insulated sheath portion 34 is therefore provided therebetween. As can be seen in Figure 2, insulated sheath portion 34 may extend over a significant length of the electrodes in order to ensure that absolutely no electrical contact can be made between the distal portions 21 of the electrodes 20 and the sheath 12, as well as to provide for a more free-flowing movement between the electrodes and sheath during their advancement/retraction within the sheath.

The inventors note that using portion 32 of the sheath 12 as a return electrode may help to negate some of the problems associated with the poor conductivity of lung tissue. If the return electrode 32 is also in contact with the tumour tissue, then ablation can occur in tissue that has a higher electrical conductivity than that of surrounding healthy lung tissue. For example, whilst conductivity values change with frequency, lung tumour tissue (inflated) is reportedly 1.6 - 2 times more conductive than healthy tissue, meaning that more effective ablations are likely to occur if both electrodes are in the tumour. Similar effects should be apparent for ablations in the liver, for example, as liver tumour tissue has been described as being 6.5-7 times more conductive than surrounding healthy tissue.

A cross sectional view of the sheath 12, taken along the line 4-4 in Figure 3, with the electrodes 20A, 20B and 20C contained within the (non-vacated) lumen 18 is shown in Figure 4. The insulated sheath 34 can clearly be seen as providing an insulative physical barrier between the sheath 12 and electrodes 20.

Referring now to Figure 5, shown is the distal end 14 of the sheath of an ablation device in accordance with another embodiment. As can be seen, the terminal end of the sheath's distal end 14 includes sharpened crenulations 36, which have a two-fold functionality. Firstly, each crenulation 36 acts to guide the distal portion 21 of a respective electrode 20 outwardly from the sheath in an equally spaced manner (i.e. at an angle of roughly 120° to one another). As would be appreciated, such an evenly spaced deployment of the electrodes 20, 20, 20 is likely to provide for a consistent ablating configuration (and hence consistent ablations), as well as significantly reduce the likelihood of the electrodes touching one another. Once the initial angle of attack of the distal tips of the electrodes 20 has been set, further advancement of the electrodes into the tissue generally results in them following the same pathway. Secondly, the crenulations 36 may also be sharpened in order to enhance the tissue penetrating ability of the sheath.

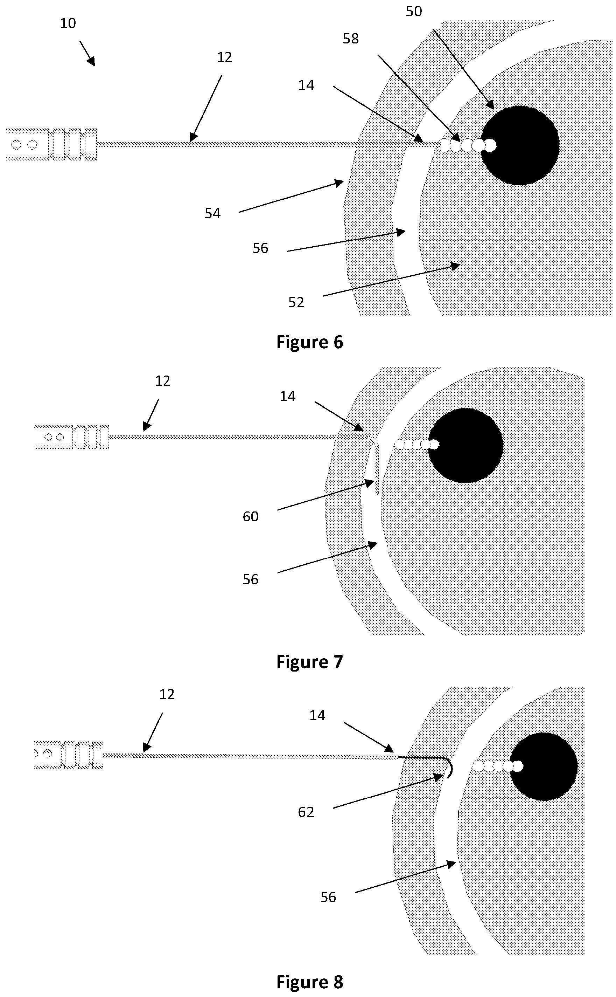

Operation of the ablation device of Figure 1 to ablate a tumour 50 in a patient's lung 52 and the post-ablation procedure will now be described with reference to Figures 6 to 8. It will be appreciated that the procedures described below could readily be adapted by a person skilled in the art to treat other tumours in other body tissues.

Firstly, the location and nature of the tumour 50 is determined, as best possible, using CT or other suitable visualisation technique. The distal end 14 of the ablation device's sheath 12 is then inserted through the patient's skin 54 and pleural cavity 56 and into the lung 52, where it can then be positioned adjacent the tumour 50. Typically, the sheath 12 would be inserted close to, but not into, the tumour 50 although, in embodiments where the distal end 14 of the sheath 12 acts as a return electrode, it may be beneficial to do so. Visualisation techniques (e.g. CT) could, for example, be employed in order to appropriately locate the tumour 50 and positon the sheath 12 in real time during its insertion. As the sheath 12 has a relatively fine gauge and hence relatively easy to control, it is less likely that the operator might accidentally mis-position the sheath, with the attendant consequences. Once so-positioned, the sheath 12 remains in the same location throughout the entirety of the ablation procedure. As would be appreciated, this is a much simpler and safer procedure than those which require multiple injections.

The electrodes 20 of the device 10 is/are then deployed into the tumour (not shown). Tissue in the lung located around the electrode (or between the electrode and sheath/grounding plate, etc.) will therefore be ablated upon application of an appropriate source of energy in a conventional manner. Typically, ablation using a generator setting of approximately 7W until no further current will pass due to impedance should be sufficient. If the ablation device is configured for multiple ablations with the electrodes being rotated between ablations in the manner described above, the electrodes would be retracted and rotated by the appropriate amount before being redeployed for the further ablation.

Once ablation is completed, the electrodes are retracted back into the ablation device 10 by a user pulling on the deployment knob, as depicted in Figure 2. The electrodes are subsequently removed, again as depicted in Figure 2 and may be discarded if they were single use. Care must now be taken because the proximal end 14 of the sheath 12 is now exposed to air and must be covered with a finger, or other plugging means, before immediately attaching a syringe (not shown) with tissue glue for track plugging.

Referring now specifically to Figure 6, shown is a tissue glue 58 (or, alternatively, a sealant or occluding device such as a balloon) being injected into the patient's lung 52 whilst, at the same time, the sheath 12 is being withdrawn out of the lung as far as the pleural cavity 56. In this manner, the track left by the sheath 12 and, more importantly, the hole between the patient's lung 52 and pleural cavity 56 should rapidly be sealed, thereby preventing, or at least reducing the likelihood of, the occurrence of complications such as pneumothorax.

Despite the best care, however, there is always a risk that the hole in the patient's lung will not be completely sealed and a pleural drain will need to be placed. Even in such circumstances, however, the ablation device of the present invention greatly simplifies the procedure. Conventionally, if it was necessary to place a pleural drain into a patient's pleural cavity, such would require further surgical intervention (e.g. a second incision), complicating the procedure and increasing the risk of post-procedure complications. Methods via which a pleural drain may be inserted into a patient's pleural cavity in accordance with embodiments of the present invention will now be described with reference to Figures 7 and 8.

A first such method is depicted in Figure 7. As shown in Figure 7, a specifically designed pleural drain 60 can be passed through the vacant lumen 18 of the sheath 12 and into the pleural space 56 via the sheath's distal end 14. Subsequently withdrawing the sheath 12 out of the patient results in the sheath being recovered (to either be discarded or retained for re-use) but with the pleural drain 60 remaining inside the patient. Although not shown, the pleural drain is then connected to a standard underwater seal bottle by means of a designated connector designed to fit the pleural drain and the chest drain bottle tubing. The drain may subsequently be removed in a conventional manner.

Figure 8 shows how a pleural drain that is larger than can be accommodated through the vacant lumen 18 of ablation device 10 may still be positioned in the plural cavity 56 via the same incision as that made for or by the sheath 12. In a first step, a guidewire 62 (or, alternatively, a flexible bougie, not shown) is placed through the sheath's vacant lumen 18 and into the pleural cavity 56. The sheath is the removed in the manner described above, leaving the guidewire 62 in place. Subsequently, a dilator or larger chest drain (not shown) can be slid over the guidewire 62 and into the pleural cavity 56, in a similar manner to the conventional "needle-wire-dilator-sheath" procedure, along the same track as that used by the sheath. Finally, the guidewire 62 can be removed and the dilator/chest drain connected to the underwater seal bottle, as described above.

These techniques represent a significant advance over conventional techniques which, as described above, usually require the surgeon to make a separate incision in the patient's chest in order to place a drain (quite probably blindly and with some urgency) and which carry attendant risks of lung/great vessel/heart/oesophagus injury

After removing the electrodes 20 from the sheath 12, these are not generally reusable. As noted above, reusing such electrodes may not be good practice because of skin puncture risks when cleaning them, and risks that the electrodes if inadequately cleaned will not perform adequately. Furthermore, the heat effects caused by ablations on smaller electrodes may adversely affect their shape and deployment characteristics, resulting in sub-optimal subsequent ablations. New electrodes may, for example, be loaded into the sheath of an ablation device using a reload device (not shown) in which the electrodes are housed in a straightener tube for ease of handling. Once the distal end(s) of the electrode(s) are located inside the lumen, the straightener tube can be removed (e.g. by a peel-apart construction, a concertina folder or by simply slipping over the deployment cable) for disposal or reuse, and the electrodes advanced into the sheath, ready for use. Alternatively, the reload device may be provided in the form of insulation sleeve 34.

As described above, relatively small tumours close to a bronchial wall are suitable for a novel approach where the tissue ablating device is part-deployed via a bronchoscope, thereby avoiding the need to puncture the pleural cavity and potentially cause pneumothorax. Such an approach also limits the length of the transpulmonary track and thus offers increased safety regarding large pulmonary blood vessels (etc.). New guidance technologies can also be used to advantage with such a bronchoscopic approach. This guidance can, for example, be in the form of real time CT, sophisticated image intersification systems producing CT like images such as the Siemens Zeego, or the specifically made pulmonary image guidance systems including Ion from the manufacturers of the Da Vinci robot (Intuitive medical).

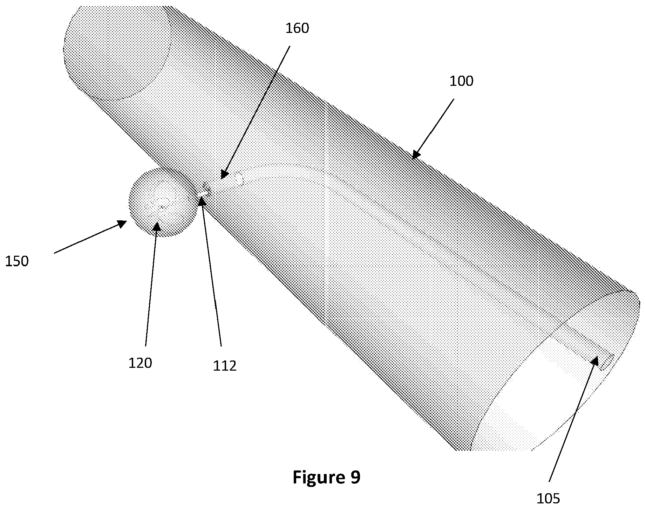

Referring now to Figure 9, shown is an embodiment of the invention suitable for performing transbronchoscopic ablations. A bronchoscope 105 is positioned within the bronchus 100 of a patient and navigated using conventional techniques. The sheath of an ablation device in accordance with an embodiment of the present invention is flexible so that it can be carried by the bronchoscope 105 through the patient's bronchus 100 into an appropriate location near a peribronchial tumour 150. Such a sheath may, for example be formed from one of the flexible materials described above and may, in some embodiments, contain a metal reinforcing mesh (not shown) which may also act as a return electrode. The sheath terminates at plug 160 which, in this embodiment, is provided by a 5mm rigid metal tube which contains the sharp ends of the electrodes 120.

In use, the plug 160 at the distal end of the ablation device's sheath is positioned adjacent the bronchial wall. Penetration of the bronchial wall may be achieved in a similar manner to that described above with respect to the percutaneous insertion of ablation device 10, where a sharpened crenulated end is caused to pierce the bronchial wall and pass through any lung tissue until it reaches the ablation site. Such configurations may, however, cause issues because the sheath needs to pan through the bronchoscope channel and the sharpened end may damage or stick to it. In alternative embodiments therefore, a monopolar diathermy cutting current may be applied to the plug 160, enabling it to cut through the bronchial wall and allow a path to the tumour. This can also be facilitated by slightly deploying the electrodes from the end of the sheath whilst pointing in the axis of the catheter to burn through the wall, followed by advancement to the ablation site/tumour.

Once in position, the electrodes 120 are deployed into the tumour 150, ideally under real time radiology control, and ablation performed as described previously. A surgical material can be passed through the vacant lumen as described above in order to seal the track and hole in the bronchial wall. As would be appreciated, the risk of complications being caused by such a treatment would be reduced compared to those involving the percutaneous insertions described above, although the position of tumour 150 with respect to the bronchus 100 will determine the suitability of this method.

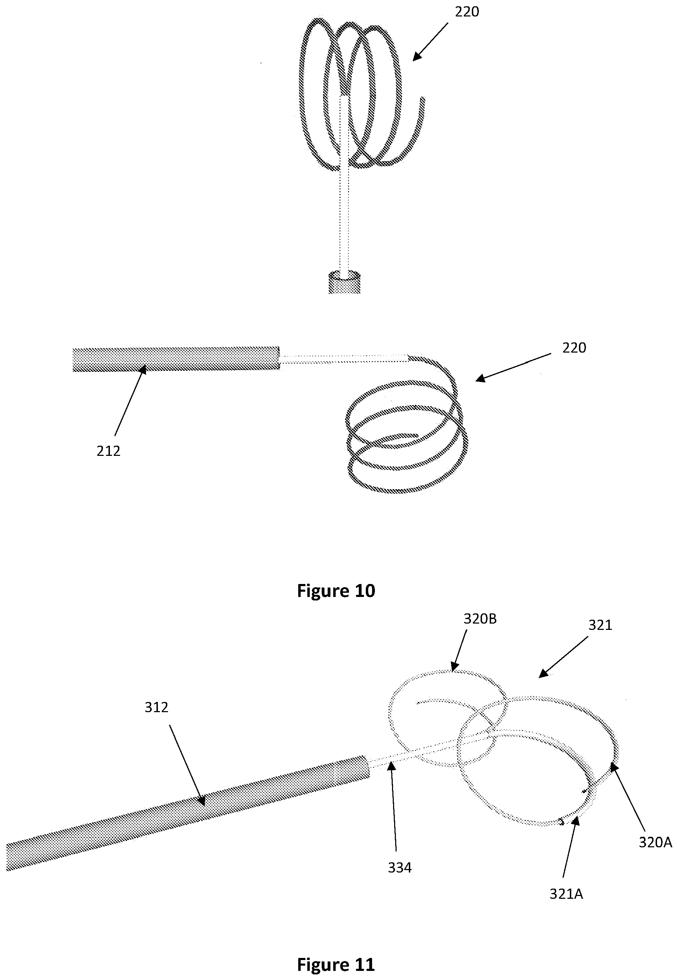

Referring now to Figures 10 and 11, alternative ablating configurations of electrodes are shown. In Figure 10, only one electrode 220 is deployed from shaft 212 in a single coil configuration. In Figure 11, helical electrodes 320A and 320B, having opposite polarities, are deployed from shaft 312. Insulation 321A is provided along a portion of the deployed electrode 320A, in order to ensure that there is no risk of the electrodes making electrical contact with one another proximal to the distal end of the sheath 312. An insulated sheath 334 is also provided to electrically separate the electrode 320 from sheath 312 (which may itself be electrically active).

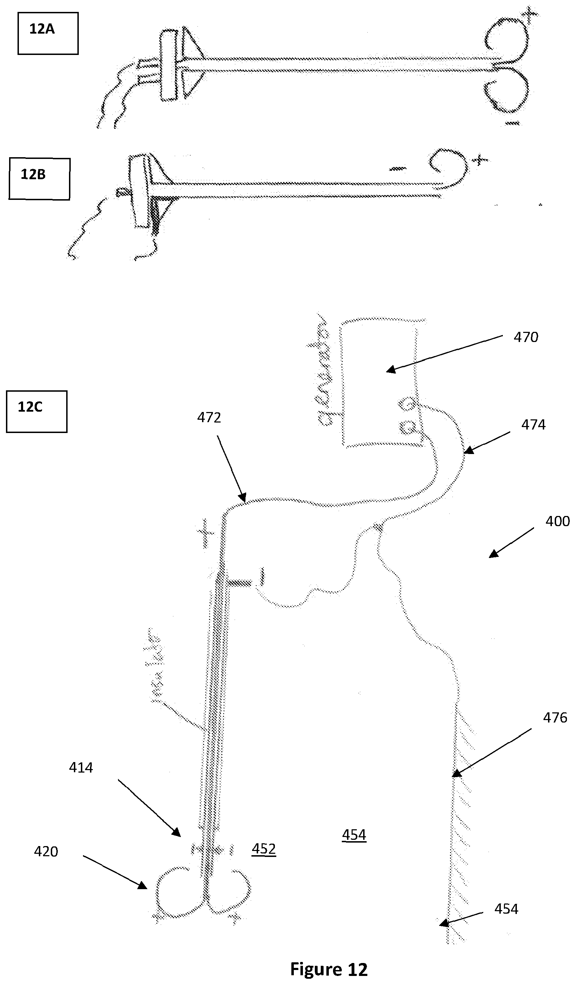

Referring now to Figures 12A and 12B, shown are ablation devices in accordance with embodiments of the present invention having two and one electrodes, respectively. The coil pusher rods and the proximal part of the electrodes are insulated so that there is no risk of an operator being exposed to an electrical current. Although not shown, the electrodes would be electrically connected to a source of energy such that, once deployed and connected to the source of energy, they can ablate tissue in the manner described herein.

Referring firstly to Figure 12A, two deployed electrode coils of opposite polarity are shown in a circular deployed configuration. The diameter of the electrode coils will vary, depending on the location and size of the desired ablation site/tumour, and may, for example, have a diameter of between about 0.5cm-2.5cm. Operation of this ablation device will ablate tissue between and around the electrodes.

Referring now to Figure 12B, shown is an ablation device in accordance with an embodiment of the present invention having a single deployed electrode coil. The electrode coil has a first polarity, and the distal end of the sheath has the opposite polarity (the sheath would be insulated on its outside down to the vicinity of the tumour). Operation of this ablation device will ablate tissue between and around the electrode and the distal end of the sheath, which may result in a different ablation to that of the ablation device of Figure 12A.

Referring now to Figure 12C, shown is an embodiment of the device of the present invention that is configured for ablating tissue concurrently in both a bipolar and monopolar manner. As described above, such operation provides multiple electric pathways for the applied current to follow, which the inventors have found can advantageously result in more predictable and consistent ablations that have relatively larger volumes than is achievable using conventional ablation devices of a similar size.

In Figure 12C, an ablation device 400 having two deployed electrode coils 420 which have an opposite polarity to that of an electrically active portion at the sheath's distal end 414 are connected to a RF generator 470. An electrical cord 472 from the positive terminal of the generator is electrically connected to the electrodes 420, whilst an electrical cord 474 from the negative terminal of the generator is electrically connected to both the sheath 414 of the ablation device and a patient grounding pad 476 that has been positioned by the operator on the patient's skin 454 at an appropriate location.

When the RF generator 470 is then operated, the synchronous provision of return paths between the deployed electrodes 420 and both the distal part of the ablation device's sheath 414 and ground pad 476 on the patient's skin cause a balance of radiofrequency delivery between these components to be established. This balance changes as tissue 452 is ablated and an impedance limit is reached. In effect, the vast majority of ablation will initially occur directly between the deployed electrodes 420 and the sheath of opposite polarity 414, these being situated relatively closely to one another. However, when the tissue 452 between the deployed electrodes 420, 420 and the sheath 414 is ablated, its conductivity decreases and the electrical field now has to work around the ablated tissue, which causes the size of the ablation to increase. As the size of the ablation increases, the proportion of the applied electrical field the device's sheath 414 will decrease, whilst the proportion of the electrical field between the electrodes 420 and the grounding pad 476 will increase, resulting in tissue 454 being ablated. Eventually, an ablation having a maximum size for the applied conditions will be formed, after which the current will be fully impeded and no further ablation will occur.

The inventors discovered that such concurrent use of the grounding pad 476 and probe sheath 414 as return electrodes can result in larger, more precise and more controllable ablations than is possible when using the sheath alone as a return electrode (i.e. as a conventional bipolar device). The inventors have found that it is not necessary to cause switching between electrodes, as is the case for some prior art devices, because the path of return of radiofrequency energy initially occurs preferentially to the sheath but, as impedance rises due to the coagulation of tissue around the sheath, the path of return of radiofrequency energy progressively passes to the patient grounding pad to return to the generator.

In effect, operation of this hybrid ablation system as described herein may advantageously be capable of effectively ablating tumours in a highly controllable manner, and even in potentially difficult to reach locations in a patient. Operation of the hybrid mono-bipolar ablation system described herein may also advantageously enable issues specific to the treatment of lung tumours to be overcome, where the presence of air (which is non-conductive) in close proximity to the tumour can affect the size and shape of the ablation. The inventors have also found that positioning of the grounding plate on the patient may be able to cause ablations having a particular shape, which may be advantageous when ablating some tumours.

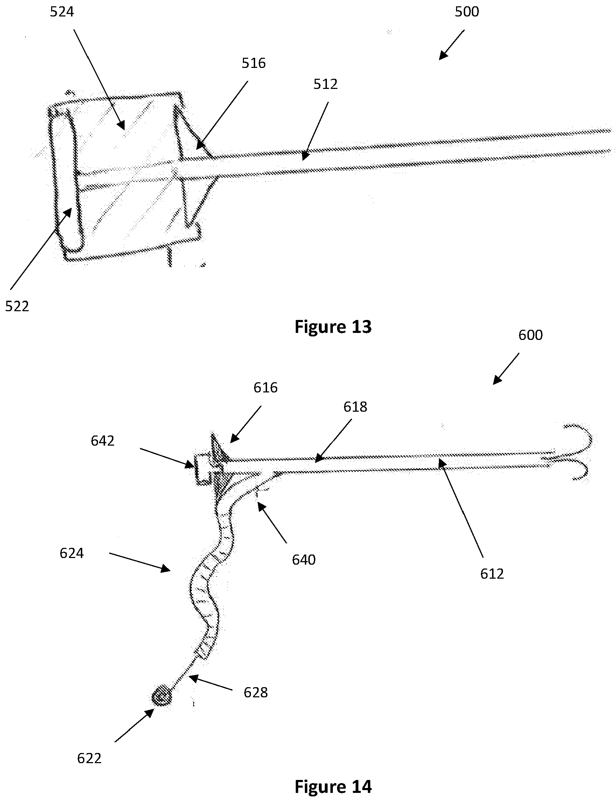

Referring now to Figures 13 and 14, embodiments of ablation devices in accordance with the present invention are shown having different handles. For reasons such as those described above, providing ablation devices having differently configured handles may help to enhance their utility. For example, the inventors recognised that more compact ablation devices would be advantageous when treating lung tumours because the limited physical space in a computed tomography (CT) machine made conventional probes (having inbuilt handles) difficult to accommodate. Such space issues may be less relevant when performing ablations in other areas of a patient's body, however, because visualisation techniques other than CT can often be used.

Referring firstly to Figure 13, shown is a handle 524 that is removably coupleable to an ablation device 500 in accordance with an embodiment of the present invention. The handle 524 is configured to fit over a housing at the proximal end 516 of the sheath 512 and the deployment knob 522, and makes manipulating the device 500 during insertion of the sheath into the patient easier. However, once this is achieved (i.e. sheath's distal end is positioned at the tumour, not shown), the handle 524 can be unclipped from the deployment knob 522 and the proximal end 516, leaving a gap which enables the deployment knob 522 to be pushed towards the sheath's proximal end 516 in order to advance and deploy the electrodes in the manner described above. This relatively simple configuration minimises the length of the device when it needs to be located in the CT machine for electrode deployment guidance purposes. In contrast, existing devices (many of which involve extremely complex assemblies) typically require that the deployment distance effectively be added to the handle length of the device.

Referring finally to Figure 14, shown is a flexible deployment handle 624 coupled to a sidewall of an ablation device 600 in accordance with another embodiment of the present invention. This configuration of device and the deployment part of a handle would also address the lack of room in a CT gantry, as described above, and may provide for a more efficient movement of the deployment cable 628 (and hence advancement and retraction of the electrode(s) within the sheath 612) than would the device of Figure 13, if the angle of the flexible deployment cable happened to be too sharp.

Instead, the flexible deployment handle 624 of Figure 14 is guided through approximately 90° by a sidearm 640 incorporated into the device's sheath 612. Advantageously, this change in angle is caused to occur within the sidearm and therefore may have a less sharp radius than may be the case for a completely flexible deployment handle. In this device, a flexible deployment sheath 624 and cable 628 of the type used in a bicycle brake (e.g. polyamide with a braided/woven stainless steel wire within the plastic moulding) may be used. The device inner cable 628 may be attached to the deployment knob 622 and an outer flexible sheath that is attached to, but removable from, the device's sheath at or about its proximal end. The device may be removed by unscrewing or unclipping to allow withdrawal of the coil electrodes in the manner described above.

A removable cap 642 is also provided to enable ready access to the lumen 618. For example, in order to inject a flowable surgical material, cap 642 is removed and a syringe inserted into the proximal end of the sheath 612.

Experiments conducted by the inventors to demonstrate the effectiveness of tissue ablation devices and methods for ablating tissue in accordance with embodiments of the present invention will now be described.

Ablations were carried out on bovine liver using the technique described below. The bovine livers were obtained fresh on the day of the experiments and were immersed in warm water at 37-40°C. The core temperature of the liver was measured with a thermocouple until 37°C was reached. After that the liver specimen was placed into a container and experiments commenced and recorded.

All ablations were performed using the RF generator's power control mode which delivers the required wattage (noted below) and ablation continued until complete tissue impedance was achieved. The time taken for full impedance to be reached was noted and the ablated liver was subsequently examined, dissected, measured and photographed. The ablated liver specimen was first bisected along the line of sight, longitudinal (x axis) and horizontal (y axis) dimensions were measured with a linear centimetre ruler and photographed. Then the specimen was transected perpendicular to the line of sight and the depth (z axis) was measured.

In a first series of experiments, an ablation device having a configuration similar to that described above in respect of Figure 12C and having two electrodes which, when deployed each define a circle having a diameter of about 2cm was inserted into a whole calf liver. Experiments were performed where the deployed electrodes were energised with 15W, with the return electrode being:
(a) an electrically conductive portion of the sheath of the device;
(b) a grounding pad positioned on a surface of the liver; and
(c) (a) and (b) in combination.

The results of these experiments are shown in the table set out below.

**Table 1 - 2 x 2cm coils - 15W**

| Return electrode | Runtime (min) | Ablation volume (cm) |
|---|---|---|
| (a) | 4.7 | 3 x 3.5 x 2 |
| (b) | 7.9 | 3 x 1 x 1 |
| (c) | 13.6 | 5 x 6 x 3 |
| (c) (repeat) | 20 | 5 x 5 x 4 |

Similar experiments were carried out using an ablation device having two electrodes which each define a circle having a diameter of about 1.5cm, with the ablation being carried out with 8W power. The results of these experiments are shown in Table 2, set out below.

**Table 2 - 2 x 1.5cm coils - 8W**

| Return electrode | Runtime (min) | Ablation volume (cm) |
|---|---|---|
| (a) | 4 | 2 x 1 x 1 |
| (b) | 8 | 2 x 3 x 2 |
| (c) | 14 | 3 x 3 x 3 |
| (c) (repeat) | 13.4 | 3 x 2 x 3 |
| (c) (repeat) | 15 | 3 x 3 x 3 |

As can clearly be seen from Tables 1 and 2, configuration (c) produces larger ablations than configurations (a) and (b), thus providing proof of concept for the hybrid monopolar/bipolar methods of the present invention described above.

In other experiments, simulations of lung tumour ablations were performed. The nature of lung tissue precludes ablation due to air insulation, and it is only when there is a solid tumour in the lung that ablation becomes possible (and is required). The inventors therefore devised experiments using lung tissue as a barrier between liver tissue and the return plate, where the piece of liver, in theory resembles a tumour in the lung. The device was tested with liver tissue embedded in lung tissue, against which the return plate was situated, to test the effect of lung's insulation properties on the ablation zone.

Successful ablations were able to be obtained using conventional monopolar RF ablation devices (abbreviated "MRFA" in the table set out below) and with ablation devices in accordance with embodiments of the present invention (abbreviated "HPRFA" in the table set out below). Representative results of these experiments are described below.

**Table 3 - Comparison between monopolar and hybrid RFA**

| Median wire caliber (mm) | Polarity | Number of experiments | Median Coil diameter (mm) | Coil wire length (mm) | Median Power (watts) | Average ablation Time (minutes) | Size (mm) | | | Volume (cc) |
|---|---|---|---|---|---|---|---|---|---|---|
| | | | | | | | x | y | z | |
| 0.3 | HPRFA | 2 | 10(5-20) | 50 | 23(15-40) | 8.55 | 30 | 32.5 | 32.5 | 132.73 |
| 0.3 | MRFA | 2 | 10(5-20) | 50 | 23(15-40) | 6.2 | 27.5 | 30 | 30.4 | 105.4 |
| 0.43 (0.4-0.45) | HPRFA | 10 | 12(8-20) | 50(42-100) | 35(25-50) | 7.42 | 32.5 | 31.5 | 31.5 | 135.08 |
| 0.43 (0.4-0.45) | MRFA | 5 | 12(8-20) | 50(42-100) | 35(25-50) | 8.4 | 31 | 28 | 34.8 | 126.53 |

As can be seen, the hybrid mono/bipolar methods of the present invention resulted in larger ablations than was the case for conventional monopolar devices.

In yet further laboratory experiments, the inventors have been able to repeatedly occlude the leakage of air from ventilated lung samples by injecting bio glue through the vacated lumen of ablation devices in accordance with the present invention.

In summary, the invention relates to tissue ablating devices for ablating biological tissue and, in particular, tumours such as lung tumours. It will be appreciated from the foregoing disclosure that the present invention provides a number of new and useful results. For example, specific embodiments of the present invention may provide one or more of the following advantages:
- the ablation device enables tumours in many locations to be ablated via a single percutaneous incision or endoscopic insertion, with subsequent steps in the procedure being accomplished using the same track or the device's *in situ* sheath;
- the vacatable sheath can be employed to deliver flowable tissue sealant/coagulant or sealant devices;
- the device and method provide for unprecedented control and precision of ablation, and can be operated to produce ablation volumes comparable with those conventionally achievable by only relatively larger devices;
- the hybrid mono-bipolar return path dependent on changing tissue impedance of particular value in high impedance tissues can enable unprecedented control and precision of ablations, even in conventionally challenging locations in the patient's body;
- ablation devices having flexible cable-like handles may be better suited for use in combination with CT imaging;
- the small gauge of the sheath enables use of the device in percutaneous procedures, lessening the complexity of the procedure and reducing possible complications; and
- choice of electrode size, shape and configuration, as well as its angle of deployment provides the operator with an unprecedented level of control over the ablation, even after the procedure has commenced;
- electrodes which can be used in the device may have a 360 degree (or greater) circular deployment, or a helix shape that provides an even greater electrode surface area.

It will be understood to persons skilled in the art of the invention that many modifications may be made without departing from the scope of the invention as defined by the claims.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

## Claims

1. A tissue ablation device (10), the device (10) comprising:
a sheath (12) comprising a distal end (14) that is positionable at an ablation site in the tissue, a proximal end (16) and a lumen (18) extending therebetween;
one or more electrodes (20) that are advanceable and retractable through the lumen, wherein a distal portion (21) of the one or more electrodes (20) is deployable into a tissue ablating configuration from the distal end (14) of the sheath (12) upon advancement, and the one or more electrodes (20) are removable from the lumen (18) via the proximal end (16) of the sheath (12) upon retraction, whereupon the lumen (18) becomes vacated;
wherein the sheath (12) is configured for a surgical material to traverse the vacated lumen (18) for delivery from the distal end (14) of the sheath (12) into the tissue;
the device (10) **characterised in that** the distal end (14) of the sheath (12) comprises crenulations (36) for guiding an initial direction of deployment of the one or more electrodes (20), and the tissue ablating configuration of the or each of the one or more electrodes (20) is independently circular or helical in shape.

2. The device (10) of claim 1, further comprising a dispenser which is operable to dispense the surgical material into the vacated lumen (18).

3. The device (10) of claim 2, wherein the dispenser is attachable to the proximal end (16) of the sheath (12).

4. The device (10, 400) of any one of claims 1 to 3, wherein the one or more electrodes (20, 420) are configured to have a polarity during ablation opposite that of a grounding pad (476) positioned on the body of a patient, whereby ablation occurs between the electrode(s) (20, 420) and the grounding pad (476).

5. The device (10, 400) of any one of claims 1 to 4, wherein the one or more electrodes (20, 420) are configured to have a polarity during ablation opposite that of an electrically conductive portion of the sheath (12, 414), whereby ablation occurs between the electrode(s) (20, 420) and the electrically conductive portion of the sheath (12, 414).

6. The device (10) of any one of claims 1 to 3, wherein the one or more electrodes (20) comprise a plurality of electrodes (320A, 320B) configured to have opposite polarities during ablation.

7. The device (10) of any one of claims 1 to 6, wherein the or each of the one or more electrodes (20) have a cross-sectional shape that is independently selected from: circular, a circular segment, elliptical, triangular and flat.

8. The device (10) of any one of claims 1 to 7, wherein the one or more electrodes (20) are configured for single use.

9. The device (10) of any one of claims 1 to 8, wherein the one or more electrodes (20) are formed from a material selected from carbon steel or a nickel-titanium alloy.

10. The device (10) of any one of claims 1 to 9, wherein the sheath (12) is configured for percutaneous insertion into the tissue or for endoscopic insertion into the tissue.

11. The device (10, 600) of any one of claims 1 to 10, further comprising a deployment handle (624) which is operable to advance and retract the one or more electrodes (20) through the lumen (18, 618), wherein the deployment handle (624) is preferably orientated at an angle to the sheath (12, 612).

## Patentansprüche

1. Vorrichtung zur Ablation von Gewebe (10), wobei die Vorrichtung (10) Folgendes umfasst:
eine Hülle (12), umfassend ein distales Ende (14), das an einer Ablationsstelle in dem Gewebe positionierbar ist, ein proximales Ende (16) und ein Lumen (18), das sich dazwischen erstreckt;
eine oder mehrere Elektroden (20), die durch das Lumen vorschiebbar und zurückziehbar sind, wobei ein distaler Abschnitt (21) der einen oder mehreren Elektroden (20) in eine gewebeablatierende Konfiguration von dem distalen Ende (14) der Hülle (12) beim Vorschieben entfaltbar ist und die eine oder mehreren Elektroden (20) aus dem Lumen (18) über das proximale Ende (16) der Hülle (12) beim Zurückziehen entfernbar sind, woraufhin das Lumen (18) frei wird;
wobei die Hülle (12) für ein chirurgisches Material konfiguriert ist, um das freie Lumen (18) zur Abgabe von dem distalen Ende (14) der Hülle (12) in das Gewebe zu durchqueren;
wobei die Vorrichtung (10) **dadurch gekennzeichnet ist, dass** das distale Ende (14) der Hülle (12) Zinnen (36) zum Führen einer anfänglichen Entfaltungsrichtung der einen oder mehreren Elektroden (20) umfasst und die gewebeablatierende Konfiguration der oder jeder von der einen oder den mehreren Elektroden (20) unabhängig kreisförmig oder schraubenförmig ist.

2. Vorrichtung (10) nach Anspruch 1, ferner umfassend einen Spender, der bedienbar ist, um das chirurgische Material in das freie Lumen (18) zu spenden.

3. Vorrichtung (10) nach Anspruch 2, wobei der Spender an dem proximalen Ende (16) der Hülle (12) anbringbar ist.

4. Vorrichtung (10, 400) nach einem der Ansprüche 1 bis 3, wobei die eine oder mehreren Elektroden (20, 420) konfiguriert sind, um eine Polarität während Ablation entgegengesetzt zu derjenigen eines Erdungspads (476) aufzuweisen, das an dem Körper eines Patienten positioniert ist, wodurch Ablation zwischen der/den Elektrode(n) (20, 420) und dem Erdungspad (476) stattfindet.

5. Vorrichtung (10, 400) nach einem der Ansprüche 1 bis 4, wobei die eine oder mehreren Elektroden (20, 420) dazu konfiguriert sind, eine Polarität während Ablation entgegengesetzt zu derjenigen eines elektrisch leitenden Abschnittes der Hülle (12, 414) aufzuweisen, wodurch Ablation zwischen der/den Elektrode(n) (20, 420) und dem elektrisch leitenden Abschnitt der Hülle (12, 414) stattfindet.

6. Vorrichtung (10) nach einem der Ansprüche 1 bis 3, wobei die eine oder mehreren Elektroden (20) eine Vielzahl von Elektroden (320A, 320B) umfassen, die dazu konfiguriert sind, entgegengesetzte Polaritäten während Ablation aufzuweisen.

7. Vorrichtung (10) nach einem der Ansprüche 1 bis 6, wobei die oder jede von der einen oder den mehreren Elektroden (20) eine Querschnittsform aufweist, die unabhängig ausgewählt ist aus: kreisförmig, einem Kreissegment, elliptisch, dreieckig und flach.

8. Vorrichtung (10) nach einem der Ansprüche 1 bis 7, wobei die eine oder mehreren Elektroden (20) zur einmaligen Verwendung konfiguriert sind.

9. Vorrichtung (10) nach einem der Ansprüche 1 bis 8, wobei die eine oder mehreren Elektroden (20) aus einem Material ausgewählt aus Kohlenstoffstahl oder einer Nickel-TitanLegierung gebildet sind.

10. Vorrichtung (10) nach einem der Ansprüche 1 bis 9, wobei die Hülle (12) zur perkutanen Einführung in das Gewebe oder zur endoskopischen Einführung in das Gewebe konfiguriert ist.

11. Vorrichtung (10, 600) nach einem der Ansprüche 1 bis 10, ferner umfassend einen Entfaltungsgriff (624), der bedienbar ist, um die eine oder mehreren Elektroden (20) durch das Lumen (18, 618) vorzuschieben und zurückzuziehen, wobei der Entfaltungsgriff (624) bevorzugt in einem Winkel zu der Hülle (12, 612) ausgerichtet ist.

## Revendications

1. Dispositif d'ablation de tissu (10), le dispositif (10) comprenant :
une gaine (12) comprenant une extrémité distale (14) qui peut être positionnée au niveau d'un site d'ablation dans le tissu, une extrémité proximale (16) et une lumière (18) s'étendant entre celles-ci ;
une ou plusieurs électrodes (20) qui peuvent être avancées et rétractées à travers la lumière, une partie distale (21) de la ou des électrodes (20) pouvant être déployée dans une configuration d'ablation de tissu à partir de l'extrémité distale (14) de la gaine (12) lors de l'avancement, et la ou les électrodes (20) pouvant être retirées de la lumière (18) par l'intermédiaire de l'extrémité proximale (16) de la gaine (12) lors de la rétraction, après quoi la lumière (18) devient libre ;
ladite gaine (12) étant configurée pour qu'un matériau chirurgical traverse la lumière (18) libérée pour une administration depuis l'extrémité distale (14) de la gaine (12) dans le tissu ;
le dispositif (10) étant **caractérisé en ce que** l'extrémité distale (14) de la gaine (12) comprend des créneaux (36) pour guider une direction initiale de déploiement de la ou des électrodes (20), et **en ce que** la configuration d'ablation de tissu de chacune de la ou des électrodes (20) est de forme circulaire ou hélicoïdale indépendamment.

2. Dispositif (10) selon la revendication 1, comprenant en outre un distributeur qui peut être utilisé pour distribuer le matériau chirurgical dans la lumière (18) libérée.

3. Dispositif (10) selon la revendication 2, ledit distributeur pouvant être fixé à l'extrémité proximale (16) de la gaine (12).

4. Dispositif (10, 400) selon l'une quelconque des revendications 1 à 3, ladite ou lesdites électrodes (20, 420) étant configurées pour présenter, pendant l'ablation, une polarité opposée à celle d'un plot de mise à la terre (476) positionné sur le corps d'un patient, moyennant quoi l'ablation se produit entre la ou les électrodes (20, 420) et le plot de mise à la terre (476).

5. Dispositif (10, 400) selon l'une quelconque des revendications 1 à 4, ladite ou lesdites électrodes (20, 420) étant configurées pour présenter, pendant l'ablation, une polarité opposée à celle d'une partie électroconductrice de la gaine (12, 414), moyennant quoi l'ablation se produit entre la ou les électrodes (20, 420) et la partie électroconductrice de la gaine (12, 414).

6. Dispositif (10) selon l'une quelconque des revendications 1 à 3, ladite ou lesdites électrodes (20) comprenant une pluralité d'électrodes (320A, 320B) configurées pour présenter des polarités opposées pendant l'ablation.

7. Dispositif (10) selon l'une quelconque des revendications 1 à 6, ladite ou chacune desdites électrodes (20) présentant une forme de section transversale qui est choisie indépendamment parmi : circulaire, un segment circulaire, elliptique, triangulaire et plate.

8. Dispositif (10) selon l'une quelconque des revendications 1 à 7, ladite ou lesdites électrodes (20) étant configurées pour un usage unique.

9. Dispositif (10) selon l'une quelconque des revendications 1 à 8, ladite ou lesdites électrodes (20) étant formées à partir d'un matériau choisi parmi l'acier au carbone ou un alliage nickel-titane.

10. Dispositif (10) selon l'une quelconque des revendications 1 à 9, ladite gaine (12) étant configurée pour une insertion percutanée dans le tissu ou pour une insertion endoscopique dans le tissu.

11. Dispositif (10, 600) selon l'une quelconque des revendications 1 à 10, comprenant en outre une poignée de déploiement (624) qui peut être actionnée pour faire avancer et rétracter la ou les électrodes (20) à travers la lumière (18, 618), ladite poignée de déploiement (624) étant de préférence orientée selon un angle par rapport à la gaine (12, 612).
